# EUROPEAN PATENT APPLICATION

(11) **EP 4 596 582 A1**
(43) Date of publication of application: **06.08.2025**
(21) Application number: 23896676.6
(22) Date of filing: 24.11.2023
(51) Int. Cl.: C07K 16/28, C07K 16/30, C12N 15/63, C12N 5/10, C12N 15/13, A61K 39/00, A61K 39/395, A61P 35/00, G01N 33/574

(54) **B7-H3 BINDING PROTEIN AND USE THEREOF**

(30) Priority: 29.11.2022 CN 202211509364; 08.12.2022 CN 202211573211
(71) Applicant: Sichuan Kelun-Biotech Biopharmaceutical Co., Ltd., Chengdu, Sichuan 611138 (CN)
(72) Inventor: LUO, Shuntao, Chengdu, Sichuan 611138 (CN); LEI, Dongmei, Chengdu, Sichuan 611138 (CN); YUAN, Xiaoxi, Chengdu, Sichuan 611138 (CN); CHEN, Mei, Chengdu, Sichuan 611138 (CN); LIU, Hanjie, Chengdu, Sichuan 611138 (CN); TAN, Miao, Chengdu, Sichuan 611138 (CN); TAN, Xiangyang, Chengdu, Sichuan 611138 (CN); GE, Junyou, Chengdu, Sichuan 611138 (CN)
(74) Representative: Meissner Bolte Partnerschaft mbB
(86) International application number: PCT/CN2023/133969
(87) International publication number: WO 2024/114525

(57) **Abstract**

The present invention relates to the field of disease treatment. In particular, the present invention relates to an anti-B7-H3 antibody or an antigen-binding fragment thereof, nucleic acid molecules encoding same, and a method for preparing same. The anti-B7-H3 antibody or the antigen-binding fragment thereof of the present invention has a high specificity and high affinity for B7-H3. The present invention further relates to the use of the antibody or the antigen-binding fragment thereof in the treatment and diagnosis of diseases.

## Description

### TECHNICAL FIELD

The present invention belongs to the field of therapeutic monoclonal antibodies and, in more particular, relates to an antibody against B7-H3 and to the use of the antibody in the treatment and diagnosis of diseases.

### BACKGROUND

B7-H3 (CD276) is a type I transmembrane protein located on human chromosome 15 with a molecular weight of 45 kD-66 kD. As one of the co-stimulatory molecules of the B7 family, B7-H3 has 20%-27% amino acid sequence identity with other family members. Structurally, B7-H3 has extracellular IgV/IgC tandem repeats, transmembrane regions, and intracellular domains (similar to PD-L1). According to the number of extracellular tandem repeats of B7-H3, two forms have been found, namely 2Ig-B7-H3 and 4Ig-B7-H3 (one more IgV/C repeat), while 4Ig-B7-H3 is considered to be the more common form. Studies have shown that matrix metalloproteinases can cleave 2Ig-B7-H3 into a serum free form. mRNA of B7-H3 is widely distributed, but no positive expression is detected in lymphatic organs, including spleen, lymph nodes, bone marrow, thymus, etc. However, B7-H3 protein is only constitutively expressed on non-immune resting fibrocytes, endothelial cells, osteoblasts and amniotic stem cells, and inductively expressed on activated T cells, NK cells, DC cells and macrophages. In normal tissues, IHC was negative in many tissues, but low to moderate antigen expression was detected in pancreas, liver, colon, stomach, placenta, skin, adrenal gland and other tissues. Studies have shown that mRNA of B7-H3 is overexpressed in a variety of tumors, such as breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, and thyroid cancer. Many studies have shown that B7-H3 plays an important role in the progression of tumors, including promoting tumor proliferation/migration, mediating EMT transformation of tumor cells, affecting metabolism of tumor cells, and so on. The expression of B7-H3 is regulated by carcinogenic genes, and the up-regulation of B7-H3 promotes tumor growth and metastasis through various signaling pathways.

Currently, there are no antibody drugs targeting B7-H3 on the market. Therefore, it is urgent and desirable to develop antibodies targeting B7-H3 with higher specificity, lower toxic and side effects, better clinical efficacy and more convenient administration, which will provide patients with more drug choices.

### SUMMARY

In the present disclosure, the inventors developed high-affinity human antibodies with excellent properties that are capable of specifically identifying/binding to B7-H3, but not binding to or substantially not binding to B7-1, B7-2, B7-H1, B7-H2 and/or B7-H4, and have no ADCC activity, effectively avoiding side effects caused by ADCC function. This resulted in the following invention.

### The Antibody of the Invention

In one respect, the present invention provides an antibody specifically binding to B7-H3 or an antigen-binding fragment thereof, wherein the antibody or the antigen-binding fragment thereof comprises the following complementary determining regions (CDRs):
(a) a CDR-H1, a CDR-H2 and a CDR-H3 contained in a heavy chain variable region (VH) as set forth in SEQ ID NO: 1; and/or a CDR-L1, CDR-L2 and CDR-L3 contained in a light chain variable region (VL) as set forth in SEQ ID NO: 2;
(b) a CDR-H1, a CDR-H2 and a CDR-H3 contained in a heavy chain variable region (VH) as set forth in SEQ ID NO: 3; and/or a CDR-L1, CDR-L2 and CDR-L3 contained in a light chain variable region (VL) as set forth in SEQ ID NO: 4; or
(c) a CDR-H1, a CDR-H2 and a CDR-H3 contained in the following heavy chain variable region (VH), and/or a CDR-L1, a CDR-L2 and a CDR-L3 contained in the following light chain variable region (VL), wherein the heavy chain variable region (VH) and/or the light chain variable region (VL) contains a mutation in at least one CDR compared to the heavy chain variable region and/or the light chain variable region described in (a) or (b), wherein the mutation is a substitution, deletion, or addition of one or more amino acids (for example, a substitution, deletion, or addition of 1, 2 or 3 amino acids).

In certain embodiments, the substitution is a conservative substitution.

In certain embodiments, the CDRs are defined according to the IMGT, Kabat, Chothia, or AbM numbering system.

In certain embodiments, the B7-H3 includes human B7-H3 and/or monkey B7-H3. In certain embodiments, the monkey refers to Macaca mulatta.

In certain embodiments, the antibody or the antigen-binding fragment thereof according to the present invention comprises a heavy chain variable region (VH) and/or a light chain variable region (VL), wherein CDRs are defined by the IMGT numbering system:
(1a) a heavy chain variable region (VH) comprising the following three CDRs: a CDR-H1 with a sequence as set forth in SEQ ID NO: 5 or a variant thereof; a CDR-H2 with a sequence as set forth in SEQ ID NO: 6 or a variant thereof; and a CDR-H3 with a sequence as set forth in SEQ ID NO: 7 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof; or
(1b) a heavy chain variable region (VH) comprising the following three CDRs: a CDR-H1 with a sequence as set forth in SEQ ID NO: 18 or a variant thereof; a CDR-H2 with a sequence as set forth in SEQ ID NO: 19 or a variant thereof; and a CDR-H3 with a sequence as set forth in SEQ ID NO: 20 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
wherein the variant in any one of (1a) and (1b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the Chothia numbering system:
(2a) a heavy chain variable region (VH) comprising the following three CDRs: a CDR-H1 with a sequence as set forth in SEQ ID NO: 11 or a variant thereof; a CDR-H2 with a sequence as set forth in SEQ ID NO: 12 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(2b) a heavy chain variable region (VH) comprising the following three CDRs: a CDR-H1 with a sequence as set forth in SEQ ID NO: 21 or a variant thereof; a CDR-H2 with a sequence as set forth in SEQ ID NO: 22 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (2a) and (2b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the Kabat numbering system:
(3a) a heavy chain variable region (VH) comprising the following three CDRs: a CDR-H1 with a sequence as set forth in SEQ ID NO: 16 or a variant thereof; a CDR-H2 with a sequence as set forth in SEQ ID NO: 17 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(3b) a heavy chain variable region (VH) comprising the following three CDRs: a CDR-H1 with a sequence as set forth in SEQ ID NO: 24 or a variant thereof; a CDR-H2 with a sequence as set forth in SEQ ID NO: 25 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (3a) and (3b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises a heavy chain variable region (VH) and/or a light chain variable region (VL), wherein CDRs are defined by the AbM numbering system:
(4a) a heavy chain variable region (VH) comprising the following three CDRs: a CDR-H1 with a sequence as set forth in SEQ ID NO: 26 or a variant thereof; a CDR-H2 with a sequence as set forth in SEQ ID NO: 27 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof; or
(4b) a heavy chain variable region (VH) comprising the following three CDRs: a CDR-H1 with a sequence as set forth in SEQ ID NO: 28 or a variant thereof; a CDR-H2 with a sequence as set forth in SEQ ID NO: 29 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (4a) and (4b) has a substitution, deletion or addition of one or more amino acids (for example, a substitution, deletion or addition of 1, 2 or 3 amino acids) compared to the sequence from which the variant is derived; preferably, the substitution is a conservative substitution.

In certain embodiments, the antibody or the antigen-binding fragment thereof according to the present invention comprises:
(a) a VH containing a sequence as set forth in SEQ ID NO: 1 or a variant thereof; and/or a VL containing a sequence as set forth in SEQ ID NO: 2 or a variant thereof; or
(b) a VH containing a sequence as set forth in SEQ ID NO: 3 or a variant thereof; and/or a VL containing a sequence as set forth in SEQ ID NO: 4 or a variant thereof;
wherein the variant has at least 80%, at least 85%, at least 90%, at least 91%, at least 92%, at least 93%, at least 94%, at least 95%, at least 96%, at least 97%, at least 98%, at least 99%, or 100% sequence identity to the sequence from which the variant is derived or has a substitution, deletion or addition of one or more amino acids (for example, a substitution, deletion or addition of 1, 2, 3, 4 or 5 amino acids) compared to the sequence from which the variant is derived; preferably, the substitution is a conservative substitution.

In certain embodiments of the antibody or the antigen-binding fragment thereof disclosed herein, the heavy chain constant domain may comprise C-terminal lysine or lack C-terminal lysine or C-terminal glycine-lysine dipeptide. In some embodiments of the antibody or the antigen-binding fragment thereof, an N-terminal amino acid of the antibody or the antigen-binding fragment thereof may be cyclized into pyroglutamic acid. In some embodiments of the antibody or the antigen-binding fragment thereof, an N-terminal amino acid of the antibody or the antigen-binding fragment thereof may be cyclized into pyroglutamic acid.

As is known to those skilled in the art, pyroglutamic acid is a conjugated acid of pyroglutamate and is in equilibrium with pyroglutamate in a solution.

In certain embodiments, compositions comprising the antibody or the antigen-binding fragment disclosed herein are provided, and various antibodies or antigen-binding fragments therein may independently comprise C-terminal lysine, lack C-terminal lysine, lack C-terminal glycine-lysine and/or comprise N-terminal glutamine or glutamic acid; the N-terminal amino acid may be cyclized into to pyroglutamic acid or the N-terminal amino acid may be cyclized into pyroglutamate.

In certain embodiments, the antibody or the antigen-binding fragment disclosed herein comprises an antibody specifically binding to an antigen or an antigen-binding fragment thereof, and may comprise a post-translational modification thereof (e.g., C-terminal lysine shear in a heavy chain) (the N-terminal glutamine or glutamic acid in a heavy chain or a light chain is converted to pyroglutamic acid or pyroglutamate), which may occur in a host cell (e.g., CHO cell) during recombinant expression or during purification/storage.

In certain embodiments, the N-terminal glutamine of the VH having the sequence as set forth in SEQ ID NO: 1 or 3 or a variant thereof undergoes cyclization to form pyroglutamic acid or pyroglutamate; and/or the N-terminal glutamic acid of the VL having the sequence as set forth in SEQ ID NO: 2 or 4 or a variant thereof undergoes cyclization to form pyroglutamic acid or pyroglutamate.

In certain embodiments, the antibody or the antigen-binding fragment thereof having any one of the properties (1a), (2a), (3a), (4a) or (a) described in the above embodiments further has properties selected from the following:
(1) binding to B7-H3 (e.g., human or monkey B7-H3) with EC50 of less than about 100 ng/mL, such as less than about 80 ng/mL, 50 ng/mL, 20 ng/mL, 15 ng/mL, 14 ng/mL, 13 ng/mL, 12 ng/mL,11 ng/mL, 10 ng/mL, 9 ng/mL, 8 ng/mL, 7 ng/mL, 6 ng/mL, 5 ng/mL, 4 ng/mL or less; preferably, the EC50 is measured by ELISA;
(2) binding to B7-H3 (e.g., human or monkey B7-H3) with KD of less than about 100 nM, such as less than about 90 nM, 80 nM, 70 nM, 60 nM, 50 nM, 40 nM, 30 nM, 20 nM, 15 nM, 10 nM, 5 nM or less; preferably, the KD is measured by Bio-Layer Interferometry (BLI) (e.g., ForteBio Octet^{®});
(3) not binding to or substantially not binding to B7-1, B7-2, B7-H1, B7-H2 and/or B7-H4, which is assayed by, for example, ELISA;
(4) having CDC, such as inducing killing of cells expressing B7-H3 (e.g., tumor cells) through CDC;
(5) not having ADCC;
(6) inducing endocytosis of B7-H3, for example, which is assayed by, for example, flow cytometry;
(7) inhibiting proliferation of a cell (e.g., a tumor cell); and/or
(8) inhibiting tumor growth.

In certain embodiments, the antibody or the antigen-binding fragment thereof having any one of the properties (1b), (2b), (3b), (4b) or (b) described in the above embodiments further has properties selected from the following:
(1) binding to B7-H3 (e.g., human or monkey B7-H3) with EC50 of less than about 100 ng/mL, such as less than about 80 ng/mL, 50 ng/mL, 20 ng/mL, 15 ng/mL, 14 ng/mL, 13 ng/mL, 12 ng/mL,11 ng/mL, 10 ng/mL, 9 ng/mL, 8 ng/mL, 7 ng/mL, 6 ng/mL, 5 n g/mL, 4 ng/mL or less; preferably, the EC50 is measured by ELISA;
(2) binding to B7-H3 (e.g., human or monkey B7-H3) with KD of less than about 100 nm, such as less than about 90 nm, 80 nm, 70 nm, 60 nm, 50 nm, 40 nm, 30 nm, 20 nm, 15 nm, 10 nm, 5 nm or less; preferably, the KD is measured by Bio-Layer Interferometry (BLI) (e.g., ForteBio Octet^{®});
(3) not binding to or substantially not binding to B7-1, B7-2, B7-H1, B7-H2 and/or B7-H4, which is assayed by, for example, ELISA;
(4) having CDC, such as inducing killing of cells expressing B7-H3 (e.g., tumor cells) through CDC;
(5) not having ADCC;
(6) inducing endocytosis of B7-H3, which is assayed by, for example, flow cytometry;
(7) inhibiting proliferation of a cell (e.g., a tumor cell); and/or
(8) inhibiting tumor growth.

In certain embodiments, the antibody or the antigen-binding fragment thereof described in any of the above embodiments may comprise a constant region from or derived from a human immunoglobulin.

In certain embodiments, a heavy chain of the antibody or the antigen-binding fragment thereof comprises a heavy chain constant region from or derived from a human immunoglobulin, such as IgG1, IgG2, IgG3 or IgG4. In certain embodiments, the heavy chain of the antibody or the antigen-binding fragment thereof comprises a wild-type Fc region, or comprises a mutated or chemically modified Fc region that has altered effector function (e.g. reduced ADCC) as compared to the wild-type Fc region. In certain exemplary embodiments, the antibody or the antigen-binding fragment thereof according to the present invention comprises a variant of the heavy chain constant region of human IgG1, wherein the variant has the following substitutions as compared to a wild-type sequence from which the variant is derived: Leu234Ala, Leu235Ala, and Gly237Ala (according to the locations in the EU numbering system). In such embodiments, the antibody or the antigen-binding fragment thereof according to the present invention has reduced ADCC. In certain embodiments, the heavy chain of the antibody or the antigen-binding fragment thereof comprises a sequence as set forth in SEQ ID NO: 30 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence. In certain embodiments, the heavy chain of the antibody or the antigen-binding fragment thereof comprises a sequence as set forth in SEQ ID NO: 31 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence.

In certain embodiments, the heavy chain constant region (CH) or a variant thereof as set forth in SEQ ID NO: 30 or 31 lacks C-terminal lysine.

In certain embodiments, a light chain of the antibody or the antigen-binding fragment thereof comprises a light chain constant region from or derived from a human immunoglobulin, such as κ or λ. In certain embodiments, the light chain of the antibody or the antigen-binding fragment thereof comprises a sequence as set forth in SEQ ID NO: 32 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to the sequence.

In certain embodiments, the antibody or the antigen-binding fragment thereof according to the present invention comprises:
(1) a heavy chain containing a VH as set forth in SEQ ID NO: 1 and a heavy chain constant region (CH) as set forth in SEQ ID NO: 30; and a light chain containing a VL as set forth in SEQ ID NO: 2 or a light chain constant region (CL) as set forth in SEQ ID NO: 32;
(2) a heavy chain containing a VH as set forth in SEQ ID NO: 3 and a heavy chain constant region (CH) as set forth in SEQ ID NO: 30; and a light chain containing a VL as set forth in SEQ ID NO: 4 or a light chain constant region (CL) as set forth in SEQ ID NO: 32;
(3) a heavy chain containing a VH as set forth in SEQ ID NO: 1 and a heavy chain constant region (CH) as set forth in SEQ ID NO: 31; and a light chain containing a VL as set forth in SEQ ID NO: 2 or a light chain constant region (CL) as set forth in SEQ ID NO: 32; or
(4) a heavy chain containing a VH as set forth in SEQ ID NO: 3 and a heavy chain constant region (CH) as set forth in SEQ ID NO: 31; and a light chain containing a VL as set forth in SEQ ID NO: 4 or a light chain constant region (CL) as set forth in SEQ ID NO: 32.

In certain embodiments, the antibody or the antigen-binding fragment thereof according to the present invention comprises:
(1) a heavy chain having a sequence as set forth in SEQ ID NO: 40 and a light chain having a sequence as set forth in SEQ ID NO: 41; or
(2) a heavy chain having a sequence as set forth in SEQ ID NO: 42 and a light chain having a sequence as set forth in SEQ ID NO: 43.

In certain embodiments, the N-terminal glutamine of the heavy chain having the sequence as set forth in SEQ ID NO: 40 or 42 or a variant thereof undergoes cyclization to form pyroglutamic acid or pyroglutamate; and/or the N-terminal glutamic acid of the light chain having the sequence as set forth in SEQ ID NO: 41 or 43 or variants thereof undergoes cyclization to form pyroglutamic acid or pyroglutamate.

In certain embodiments, the antibody or the antigen-binding fragment thereof according to any one of the above embodiments is a murine antibody, a chimeric antibody, a humanized antibody, or a human antibody.

In certain embodiments, the variable region of the antibody or the antigen-binding fragment thereof according to any one of the above embodiments is a human variable region.

In certain embodiments, the antibody or antigen-binding fragment thereof according to any one of the above embodiments is selected from the group consisting of ScFv, Fab, Fab ', F(ab')2, Fab'-SH, Fv fragment, disulfide-stabilized Fv(dsFv), diabody, bispecific antibody, and multispecific antibody.

In certain embodiments, the antibody or the antigen-binding fragment thereof according to any one of the above embodiments carries a label. In some embodiments, the antibody or the antigen-binding fragment thereof carries a detectable label, such as an enzyme (e.g., horseradish peroxidase), radionuclide, fluorescent dye, luminescent substance (e.g., chemiluminescent substance), or biotin.

The present invention further provides the use of the antibody or the antigen-binding fragment thereof provided therein or a pharmaceutical composition thereof in tumor treatment.

### Antibody Derivatives

The antibody or the antigen-binding fragment thereof according to the present invention may be derivatized, for example, by being linked to another molecule (e.g., another polypeptide or protein). Typically, derivatization (e.g., labeling) of the antibody or the antigen-binding fragment thereof does not adversely affect its binding to B7-H3 (particularly human B7-H3). Thus, the antibody or the antigen-binding fragment thereof according to the present invention also intends to cover such derivative forms. For example, the antibody or the antigen-binding fragment thereof according to the present invention may be functionally linked (by chemical conjugation, gene fusion, non-covalently or other means) to one or more other molecular groups, for example, another antibody (for example, to form a bispecific antibody), a detection reagent, a pharmaceutical reagent, and/or a protein or polypeptide (e.g., an avidin or a polyhistidine label) capable of mediating the binding of the antibody or the antigen-binding fragment to another molecule.

As one of the derivatives of the antibody, the present invention provides a conjugate, comprising the antibody or the antigen-binding fragment thereof according to the present invention and a conjugating moiety.

In certain embodiments, the conjugating moiety is selected from detectable labels. The detectable label of the present invention may be any substance detectable by fluorescent, spectral, photochemical, biochemical, immunological, electrical, optical or chemical means. Such labels are well known in the art and the examples of the labels include, but are not limited to, enzymes (such as horseradish peroxidase, alkaline phosphatase, β-galactosidase, urease, and glucose oxidase), radionuclides (such as 3H, 125I, 35S, 14C or 32P), fluorescent dyes (such as fluorescein isothiocyanate (FITC), fluorescein, tetramethyl rhodamine isothiocyanate (TRITC), phycoerythrin (PE), Texas red, rhodamine, quantum dots or cyanine dye derivatives (such as Cy7 and Alexa 750)), acridine ester compounds, magnetic beads (e.g., Dynabeads^{®}), calorimetric labels such as colloidal gold or colored glass or plastics (such as polystyrene, polypropylene and latex) beads, and biotin used to bind to avidins (e.g. streptavidin) modified by the above labels. In certain embodiments, such labels may be useful in immunoassays (such as, enzyme-linked immunoassay, radioimmunoassay, fluorescence immunoassay, chemiluminescence immunoassay). In certain embodiments, the detectable label is selected from the group consisting of a radioisotope, a fluorescent substance, a luminescent substance, a colored substance, or an enzyme. In certain embodiments, the detectable labels described above can be linked to the antibody or the antigen-binding fragment thereof according to the present invention via linkers of different lengths to reduce potential steric hindrance.

In certain embodiments, the conjugating moiety is selected from therapeutic agents. In certain embodiments, the therapeutic agent is preferably an antitumor agent, such as a cytotoxic agent, a cytokine, a cytotoxin, or a radionuclide.

In certain embodiments, the conjugating moiety is selected from substances capable of improving the biological properties of the antibody (such as increasing the serum half-life), and the conjugating moiety may be, for example, a chemical group, such as polyethylene glycol (PEG), methyl or ethyl, or glycosyl.

As one of the derivatives of the antibody, the present invention provides a multispecific antibody comprising the antibody or the fragment thereof according to the present invention.

In certain embodiments, the multispecific antibody comprises the antibody or the antigen-binding fragment thereof according to the present disclosure as a first antigen-binding domain, and also comprises at least one second antigen-binding domain for other targets.

In certain embodiments, the antigen-binding domains of the multispecific antibody maintain their respective primary binding specificity.

In certain embodiments, the multispecific antibody is a bispecific antibody or a tri-specific antibody or a quad-specific antibody.

As one of the derivatives of the antibody, the present invention provides a chimeric antigen receptor comprising the antibody or the antigen-binding fragment thereof according to the present invention. In certain embodiments, the chimeric antigen receptor comprises the antibody or the antigen-binding fragment thereof (e.g., ScFv) according to the present invention as an extracellular antigen-binding domain that specifically binds to B7-H3, as well as a transmembrane domain and one or more intracellular T-cell signaling domains. The present invention further provides a host cell (e.g., an immune cell, such as T lymphocyte, NK cell, DC cell, and macrophage) comprising or expressing the chimeric antigen receptor.

### Antibody Preparation

The antibody of the present invention can be prepared by various methods known in the art, such as genetic engineering recombinant technology. For example, a DNA molecule encoding heavy and light chain genes of the antibody of the present invention is obtained by chemical synthesis or PCR amplification. The obtained DNA molecule is inserted into an expression vector, followed by transfecting the host cell. Then, the transfected host cell is cultured under specific conditions to express the antibody of the present invention.

The antigen-binding fragments of the present invention can be obtained by hydrolyzing an intact antibody molecule (see Morimoto et al., J. Biochem. Biophys. Methods 24:107-117

(1992) and Brennan et al., Science 229:81 (1985)). In addition, these antigen-binding fragments can also be produced directly by recombinant host cells (reviewed in Hudson, Curr. Opin. Immunol. 11: 548-557 (1999); Little et al., Immunol. Today, 21: 364-370 (2000)). For example, a Fab' fragment can be obtained directly from a host cell. Fab' fragments can be chemically conjugated to form a F(ab')₂ fragment (Carter et al., Bio/Technology, 10: 163-167 (1992)). In addition, the Fv, Fab or F(ab')₂ fragment may also be isolated directly from the culture of recombinant host cells. Other technologies for preparing these antigen-binding fragments are well known to those of ordinary skill in the art.

Thus, in another aspect, the present invention provides an isolated nucleic acid molecule comprising a nucleotide sequence encoding the antibody or the antigen-binding fragment thereof according to the present invention, or a heavy chain variable region and/or a light chain variable region thereof. In view of codon degeneracy known in the art, in certain embodiments, the nucleotide sequence may be replaceable according to codon degeneracy. In certain embodiments, the nucleotide sequence is codon-optimized.

In some embodiments, the isolated nucleic acid molecule comprises a nucleic acid molecule encoding the VH of the antibody, and/or a nucleic acid molecule encoding the VL of the antibody, wherein the nucleic acid molecule encoding the VH of the antibody comprises: (i) a nucleotide sequence as set forth in SEQ ID NO: 33, (ii) a sequence substantially identical to SEQ ID NO: 33 (e.g., a sequence having at least about 85%, 90%, 95%, 99% or higher sequence identity, or a sequence with one or more nucleotide substitutions, as compared to SEQ ID NO: 33), or (iii) a degenerate sequence of the sequence in (i) or (ii); and/or, the nucleic acid molecule encoding the VL of the antibody comprises: (iv) a nucleotide sequence as set forth in SEQ ID NO: 34, (v) a sequence substantially identical to SEQ ID NO: 34 (e.g., a sequence having at least about 85%, 90%, 95%, 99% or higher sequence identity or a sequence having one or more nucleotide substitutions, as compared to SEQ ID NO: 34), or (vi) a degenerate sequence of the sequence in (iv) or (v).

In some embodiments, the isolated nucleic acid molecule comprises a nucleic acid molecule encoding the VH of the antibody, and/or a nucleic acid molecule encoding the VL of the antibody, wherein the nucleic acid molecule encoding the VH of the antibody comprises: (i) a nucleotide sequence as set forth in SEQ ID NO: 35, (ii) a sequence substantially identical to SEQ ID NO: 35 (e.g., a sequence having at least about 85%, 90%, 95%, 99% or higher sequence identity, or a sequence with one or more nucleotide substitutions, as compared to SEQ ID NO: 35), or (iii) a degenerate sequence of the sequence in (i) or (ii); and/or, the nucleic acid molecule encoding the VL of the antibody comprises: (iv) a nucleotide sequence as set forth in SEQ ID NO: 36, (v) a sequence substantially identical to SEQ ID NO: 36 (e.g., a sequence having at least about 85%, 90%, 95%, 99% or higher sequence identity or a sequence having one or more nucleotide substitutions, as compared to SEQ ID NO: 36), or (vi) a degenerate sequence of the sequence in (iv) or (v).

In another aspect, the present invention provides a vector (such as a cloning vector or an expression vector), which comprises the isolated nucleic acid molecule according to the present invention. In certain embodiments, the vector of the present invention is, such as, a plasmid, a cosmid, a phage or a lentivirus. In certain embodiments, the vector is capable of expressing the antibody or the antigen-binding fragment thereof according to the present invention in vivo in a subject (e.g., a mammal, such as human).

In certain embodiments, the vector comprises a first nucleotide sequence encoding the heavy chain or VH of the antibody or the antigen-binding fragment thereof according to the present invention and a second nucleotide sequence encoding the light chain or VL of the antibody or the antigen-binding fragment thereof, wherein the first nucleotide sequence and the second nucleotide sequence are present on the same or different vectors. When the first nucleotide sequence and the second nucleotide sequence are present on different vectors, the vector of the present invention comprises a first vector containing the first nucleotide sequence and a second vector containing the second nucleotide sequence.

In certain embodiments, the antibody or the antigen-binding fragment thereof according to the present invention may be used to construct a chimeric antigen receptor (CAR), wherein the CAR comprises an extracellular antigen-binding domain (e.g., ScFv) that specifically binds to B7-H3, a transmembrane domain, and one or more intracellular T cell signaling domains. In such embodiments, the isolated nucleic acid molecule of the present invention may comprise a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention. In certain embodiments, the isolated nucleic acid molecule of the present invention encodes a chimeric antigen receptor comprising the antigen-binding fragment (e.g., ScFv) of the antibody according to the present invention.

In certain embodiments, the antibody or the antigen-binding fragment thereof according to the present invention may be used to construct a chimeric antigen receptor-modified immune cell, wherein the chimeric antigen receptor-modified immune cell comprises a CAR and an immune cell (such as T lymphocyte, NK cell, DC cell and macrophage).

In another aspect, the present invention provides a host cell comprising the isolated nucleic acid molecule according to the present invention or the vector according to the present invention. The host cell may be a eukaryotic cell (such as a mammalian cell, an insect cell and a yeast cell) or a prokaryotic cell (such as Escherichia coli). Suitable eukaryotic cells include but are not limited to NS0 cell, Vero cell, Hela cell, COS cell, CHO cell, ExpiCHO cell, HEK293 cell, Expi293 cell, BHK cell, and MDCKII cell. Suitable insect cells include but are not limited to Sf9 cell. In certain embodiments, the host cell of the present invention is a mammalian cell, for example, CHO (such as CHO K1, CHO-S, CHO DXB11, ExpiCHO, CHO DG44, and CHO-EBNA).

In certain embodiments, the host cell of the present invention may be a chimeric antigen receptor T cell (CAR-T). In such embodiments, the isolated nucleic acid molecule contained in the host cell may comprise a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention. In certain embodiments, the isolated nucleic acid molecule contained in the host cell encodes a chimeric antigen receptor containing an antigen-binding fragment (e.g., ScFv) of the antibody of the invention.

In another aspect, the present invention provides a method for preparing the antibody or the antigen-binding fragment thereof according to the present invention, comprising: under conditions that allow the expression of the antibody or the antigen-binding fragment thereof, culturing the host cell of the present invention, and harvesting the antibody or the antigen-binding fragment thereof from the culture of the host cell.

### Therapeutic Application

In another aspect, the present invention provides a pharmaceutical composition comprising the antibody or the antigen-binding fragment thereof, the nucleic acid, the vector, the host cell, the conjugate, the multispecific antibody, or the chimeric antigen receptor or a host cell expressing the chimeric antigen receptor according to the present invention, and a pharmaceutically acceptable carrier and/or excipient.

In certain embodiments, the pharmaceutical composition according to the present invention comprises the antibody or the antigen-binding fragment thereof according to the present invention, and a pharmaceutically acceptable carrier and/or excipient.

In certain embodiments, the pharmaceutical composition according to the present invention comprises the vector or host cell according to the present invention and a pharmaceutically acceptable carrier and/or excipient. In such embodiments, the isolated nucleic acid molecule contained in the vector comprises a nucleotide sequence encoding a chimeric antigen receptor, wherein the nucleotide sequence encoding a chimeric antigen receptor further comprises a nucleotide sequence encoding the antibody or the antigen-binding fragment (e.g., ScFv) thereof according to the present invention; the host cell comprises the isolated nucleic acid molecule or vector described above. In certain embodiments, the isolated nucleic acid molecule encodes a chimeric antigen receptor comprising the antigen-binding fragment (e.g., ScFv) of the antibody according to the present invention. In certain embodiments, the host cell is an immune cell, such as a T cell. In certain embodiments, the host cell is a chimeric antigen receptor T cell (CAR-T).

In certain embodiments, the pharmaceutical composition may also comprise an additional pharmaceutical active agent. In certain embodiments, the additional pharmaceutical active agent is a medicament with antitumor activity. In certain embodiments, the additional pharmaceutical active agent is selected from the group consisting of a B7-H3 inhibitor, an EGFR inhibitor, a HER2 inhibitor, a HER3 inhibitor, a HER4 inhibitor, an IGFR-1 inhibitor, an mTOR inhibitor, a PI3 kinase inhibitor, a c-met or VEGF inhibitor, a chemotherapeutic drug, or any combination thereof. In certain embodiments, the antibody or the antigen-binding fragment thereof according to the present invention and the additional pharmaceutical active agent are provided alone or in combination. Therefore, the antibody or the antigen-binding fragment according to the present invention and the additional pharmaceutical active agent may be administered simultaneously, separately or successively.

In certain embodiments, the antibody or the antigen-binding fragment thereof, the nucleic acid molecule, the vector, the host cell, the conjugate, the multispecific antibody, or the chimeric antigen receptor or the host cell expressing the chimeric antigen receptor in the pharmaceutical composition of the present invention is sufficient (e.g., in a subject) to:
(a) inhibit the proliferation of cells (such as tumor cells);
(b) inhibit tumor growth;
(c) induce and/or increase antibody-dependent cytotoxicity;
(d) inhibit B7-H3-mediated signaling;
(e) prevent and/or treat a B7-H3-mediated disease/disorder; or
(f) achieve any combination of (a)-(e).

In certain embodiments, the B7-H3-mediated disease/disorder is a tumor, such as a tumor expressing B7-H3. In certain embodiments, the tumor is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

In another aspect, the present invention provides use of the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition in preparation of a medicament for: inhibiting proliferation of a cell, or for prevention and/or treatment and/or adjuvant therapy of a tumor.

In certain embodiments, the medicament is used for inhibiting the proliferation of cells expressing B7-H3, such as tumor cells.

In another aspect, the present invention provides a method for inhibiting proliferation of a cell, comprising: contacting the cell with the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention. In certain embodiments, the cell is a cell expressing B7-H3, such as a tumor cell.

In another aspect, the present invention provides a method for prevention and/or treatment and/or adjuvant therapy of a tumor in a subject. The method includes administration of an effective amount of the antibody or the antigen-binding fragment thereof, the nucleic acid molecule, the vector, the host cell, the conjugate, the multispecific antibody, the chimeric antigen receptor or the host cell expressing the chimeric antigen receptor or the pharmaceutical composition according to the present invention to a subject in need.

In certain embodiments, the method further comprises the administration of a second therapy to the subject, wherein the second therapy is selected from the group consisting of surgery, chemotherapy, radiotherapy, immunotherapy, gene therapy, DNA therapy, RNA therapy, nanotherapy, viral therapy, adjuvant therapy and any combination thereof. In certain embodiments, the second therapy may be applied simultaneously, separately or sequentially with the method described above.

In any of the above embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates may be of any tumor type. In certain embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates is a B7-H3-positive tumor. In certain embodiments, the tumor to which the antibody or the antigen-binding fragment thereof, the nucleic acid molecule, the vector, the host cell, the conjugate, the multispecific antibody, the chimeric antigen receptor or the host cell expressing the chimeric antigen receptor, or the pharmaceutical composition according to the present invention relates is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

The antibody or the antigen-binding fragment thereof or the pharmaceutical composition of the invention can be formulated into any dosage form known in the medicine field, such as tablets, pills, suspensions, emulsions, solutions, gels, capsules, powders, granules, elixirs, lozenges, suppositories, injections (comprising injection solutions, sterile powders for injection, and concentrated injection solutions), inhalants, and sprays. The preferred dosage form depends on the intended administration route and the treatment use. The pharmaceutical composition of the present invention should be sterile and stable under production and storage conditions. A preferred dosage form is an injection. Such injections may be sterile injection solutions. For example, a sterile injection solution may be prepared by incorporating a necessary dose of the antibody of the present invention into an appropriate solvent, and optionally, simultaneously together with other desired ingredients (including, but not limited to, pH regulators, surfactants, adjuvants, ionic strength enhancers, isotonic agents, preservatives, diluents, or any combination thereof), followed by filtration for removal of bacteria. In addition, the sterile injection solution may be prepared into a sterile lyophilized powder (for example, by vacuum drying or freeze-drying) for easy storage and use. The sterile lyophilized powder may be dispersed in a suitable carrier, such as sterile and apyrogenic water, prior to use.

In addition, the antibody or the antigen-binding fragment thereof according to the present invention may be present in a unit dose form in the pharmaceutical composition for easy administration.

The antibody or the antigen-binding fragment thereof or the pharmaceutical composition according to the present invention may be administered by any suitable method known in the art, including, but not limited to, oral, buccal, sublingual, eyeball, topical, parenteral, rectal, intravagina, intracytoplasmic reticulum, groin, intravesical, topical (such as powder, ointment or drops), or nasal route. However, for many therapeutic uses, the preferred route/mode of administration is parenteral administration (such as intravenous, subcutaneous, intraperitoneal, and intramuscular injection). Those skilled in the art should understand that the route and/or mode of administration will change according to the intended purpose. In a preferred embodiment, the antibody or the antigen-binding fragment thereof or the pharmaceutical composition of the present invention is administrated by intravenous infusion or injection.

The pharmaceutical composition according to the present invention may comprise a "therapeutically effective amount" or "prophylactically effective amount" of the antibody or the antigen-binding fragment thereof according to the present invention. The "prophylactically effective amount" means an amount sufficient to prevent, stop, or delay the occurrence of a disease. The "therapeutically effective amount" means an amount sufficient to cure or at least partially prevent a disease and its complications in a patient suffering from the disease. The therapeutically effective amount of the antibody or the antigen-binding fragment thereof according to the present invention may vary according to the following factors: the severity of the disease to be treated, the general state of the patient's own immune system, the general condition of the patient such as age, body weight and gender, route of drug administration, and other treatments applied concurrently, etc.

In the present invention, the dosage regimen may be adjusted to obtain an optimal purpose response (e.g., therapeutic or prophylactic response). For example, a single dose may be administered, multiple doses may be administered over a period of time, or the dose may be reduced or increased proportionally with the urgency of the treatment situation.

In the present invention, the subject may be a mammal, such as a human.

### Detection Application

The antibody or the antigen-binding fragment thereof according to the present invention is capable of binding to B7-H3, thereby being useful in detecting the presence or level of B7-H3 in a sample.

Thus, in another aspect, the present invention provides a kit comprising the antibody or the antigen-binding fragment thereof according to the present invention. In certain embodiments, the antibody or the antigen-binding fragment thereof according to the invention carries a detectable label. In a preferred embodiment, the kit further comprises a secondary antibody that specifically identifies the antibody or the antigen-binding fragment thereof according to the present invention. Preferably, the secondary antibody further comprises a detectable label.

The detectable label of the present invention may be any substance detectable by fluorescent, spectral, photochemical, biochemical, immunological, electrical, optical or chemical means. Especially preferred is that such labels may be useful in immunoassays (such as, enzyme-linked immunoassay, radioimmunoassay, fluorescence immunoassay, chemiluminescence immunoassay). Such labels are well known in the art and the examples of the labels include, but are not limited to, enzymes (such as horseradish peroxidase, alkaline phosphatase, β-galactosidase, urease, and glucose oxidase), radionuclides (such as 3H, 125I, 35S, 14C or 32P), fluorescent dyes (such as fluorescein isothiocyanate (FITC), fluorescein, tetramethyl rhodamine isothiocyanate (TRITC), phycoerythrin (PE), Texas red, rhodamine, quantum dots or cyanine dye derivatives (such as Cy7 and Alexa 750)), acridine ester compounds, magnetic beads (e.g., Dynabeads^{®}), calorimetric labels such as colloidal gold or colored glass or plastics (such as polystyrene, polypropylene and latex) beads, and biotin used to bind to avidins (e.g. streptavidin) modified by the above labels. In certain embodiments, the detectable labels described above can be linked to the antibody according to the invention via linkers of different lengths to reduce potential steric hindrance.

In another aspect, the present invention provides a method for detecting the presence or level of B7-H3 in a sample, comprising a step of employing the antibody or the antigen-binding fragment thereof according to the present invention. In a preferred embodiment, the antibody or the antigen-binding fragment thereof according to the present invention further carries a detectable label. In another preferred embodiment, the method further comprises detecting the antibody or the antigen-binding fragment thereof according to the present invention using a reagent with a detectable label. The method may be used for diagnostic or non-diagnostic purpose (for example, the sample is a cell sample, instead of a sample from a patient).

In certain embodiments, the method comprises: contacting the sample with the antibody or the antigen-binding fragment thereof according to the present invention under conditions allowing formation of a complex of the antibody or the antigen-binding fragment thereof and B7-H3; and detecting the formation of the complex.

Given the low or no expression of B7-H3 in normal tissues and the expression or high expression in some cancers, a tumor may be diagnosed by detecting the presence or level of B7-H3 in a sample. Thus, in some embodiments, the method is used for diagnosing a tumor, for example, a B7-H3-positive tumor, such as breast cancer, stomach cancer, lung cancer (such as non-small cell lung cancer), colorectal cancer, pancreatic cancer, head and neck squamous cell carcinoma, melanoma, ovarian cancer, prostate cancer, liver cancer, kidney cancer, bladder cancer or any combination thereof.

In certain embodiments, the method comprises: detecting the expression level of B7-H3 in a sample to be tested from a subject; and comparing the expression level to a reference value (e.g., a healthy control), wherein an increased expression level compared to the reference value is indicative of a tumor.

In another aspect, the present invention provides the use of the antibody or the antigen-binding fragment thereof according to the present invention in preparation of a kit, wherein the kit is used for detecting the presence or level of B7-H3 in a sample and/or for diagnosing a tumor.

In another aspect, the present invention provides a diagnostic or therapeutic kit comprising the antibody or the antigen-binding fragment thereof, the nucleic acid molecule, the vector, the host cell, the conjugate, or the multispecific antibody according to the present invention, and an instruction for use. The kit may also contain a drug administration device for topical administration. The drug administration device comprises a pre-filled syringe or a needle-free device.

The antibody of the present invention has high binding affinity to B7-H3 and strong specificity, does not bind to or basically does not bind to B7-1, B7-2, B7-H1, B7-H2 and/or B7-H4, and has no ADCC, effectively avoiding side reactions caused by the function of ADCC. The antibody according to the present invention can inhibit the proliferation of tumor cells. Therefore, the antibody of the present invention has the potential to be used for the prevention and/or treatment of a tumor. In addition, the antibody according to the present invention is a human antibody and can be safely administered to human subjects without causing an immunogenic reaction. Therefore, the antibody according to the present invention is of great clinical value.

### Abbreviations

- CDR: Complementary determining region in the variable region of an immunoglobulin
- FR: Antibody framework region: amino acid residues, other than CDR residues, in the variable region of an antibody
- VH: Heavy chain variable region of an antibody
- VL: Light chain variable region of an antibody
- IgG: Immunoglobulin G
- IMGT: A numbering system based on the international ImMunoGeneTics information System^{®} (IMGT) initiated by Lefranc et al., see Lefranc et al., Dev. Comparat. Immunol. 27: 55-77, 2003.
- Kabat: Immunoglobulin alignment and numbering system proposed by Elvin A. Kabat (see, for example, Kabat et al., Sequences of Proteins of Immunological Interest, 5th Ed. Public Health Service, National Institutions of Health, Bethesda, Md., 1991).
- Chothia: Immunoglobulin numbering system proposed by Chothia et al., which is a classical rule for identifying CDR boundaries based on the location of structural ring regions (see, for example, Chothia & Lesk (1987) J. Mol. Biol. 196: 901-917; Chothia et al. (1989) Nature 342: 878-883).
- AbM: The definition of CDR comes from Martin's study (Martin ACR, Cheetham JC, Rees AR (1989) Modelling antibody hypervariable loops: A combined algorithm. Proc Natl Acad Sci USA 86: 9268-9272).
- mAb: Monoclonal antibody
- EC50: A concentration that produces 50% efficacy or binding
- IC50: A concentration that produces 50% inhibition
- ELISA: Enzyme-linked immunosorbent assay
- PCR: Polymerase chain reaction
- HRP: Horseradish peroxidase
- K_{D}: Dissociation equilibrium constant
- Ka: Association rate constant
- Kd: Dissociation rate constant
- ADCC: Antibody-dependent cell-mediated cytotoxicity
- CDC: Complement-dependent cytotoxicity
- FACS: Fluorescence activated Cell Sorting
- CDR-H1: Complementary determining region 1 in heavy variable region of immunoglobulin
- CDR-H2: Complementary determining region 2 in heavy variable region of immunoglobulin
- CDR-H3: Complementary determining region 3 in heavy variable region of immunoglobulin
- CDR-L1: Complementary determining region 1 in light variable region of immunoglobulin
- CDR-L2: Complementary determining region 2 in light variable region of immunoglobulin
- CDR-L3: Complementary determining region 3 in light variable region of immunoglobulin

### Definitions

In the invention, unless otherwise stated, the scientific and technical terms used herein have meanings commonly understood by those skilled in the art. In addition, the cell culture, biochemistry, nucleic acid chemistry, immunology laboratory and other operation steps used herein are conventional steps widely used in corresponding fields. Meanwhile, in order to better understand the invention, definitions and explanations of related terms are provided below.

As used herein, the term "antibody" is used in the broadest sense to include a variety of antibody structures, including but not limited to monoclonal antibodies, polyclonal antibodies, multispecific antibodies (e.g., bispecific antibodies), and antibody fragments, provided they exhibit the desired antigen-binding activity. For example, the antibody is an immunoglobulin molecule that consists of two pairs of polypeptide chains, each having a light chain (LC) and a heavy chain (HC). Light chains of the antibody are classified as either kappa (κ) or lambda (λ) light chains. Heavy chains can be classified as µ, δ, γ, α, or ε, and the isotypes of the antibody can be defined as IgM, IgD, IgG, IgA and IgE, respectively. Within light and heavy chains, variable and constant regions are joined by a "J" segment of about 12 or more amino acids, the heavy chain also comprising a "D" segment of about 3 or more amino acids. Each heavy chain consists of a heavy chain variable region (VH) and a heavy chain constant region (CH). The heavy chain constant region consists of three domains (CH1, CH2 and CH3). Each light chain consists of a light chain variable region (VL) and a light chain constant region (CL). The light chain constant region consists of one domain CL. The constant domains are not directly involved in the binding of antibodies and antigens, but exhibit a variety of effector functions, such as mediating the binding of immunoglobulins to host tissues or factors, including various cells (for example, effector cells) of immune system and the first component (C1q) of classical complement system. The VH and VL regions can also be subdivided into hypervariable regions (called complementary determining regions (CDRs)) interspersed with relatively conservative regions called framework regions (FR). The VH and VL regions can also be subdivided into hypervariable regions (called complementary determining regions (CDRs)) interspersed with relatively conservative regions called framework regions (FR). Each VH and VL consists of three CDRs and four FRs arranged from amino terminal to carboxyl terminal in the following order: FR1, CDR1, FR2, CDR2, FR3, CDR3, FR4. The variable regions (VH and VL) of each heavy chain/light chain pair form antigen-binding sites, respectively. The distribution of amino acids in various regions or domains can follow the definitions given by Kabat, Sequences of Proteins of Immunological Interest (National Institutes of Health, Bethesda, Md. (1987 and 1991), or Chothia & Lesk (1987) J. Mol. Biol. 196:901-917; Chothia et al. (1989) Nature 342: 878-883.

Herein, unless clearly indicated otherwise in the context, reference to the term "antibody" includes not only an intact antibody but also an antigen-binding fragment of the antibody.

The term "antibody" also includes embodiments in which the heavy chain constant region contains C-terminal lysine, or lacks C-terminal lysine or C-terminal glycine-lysine dipeptides. The term also includes embodiments in which the N-terminal amino acid of the antibody variable region has been cyclized into pyroglutamate. Thus, in compositions containing antibodies disclosed herein, the various antibodies therein may independently comprise C-terminal lysine, lack C-terminal lysine, lack C-terminal glycine-lysine and/or comprise N-terminal glutamine or glutamic acid or have N-terminal amino acid cyclized into pyroglutamic acid.

As used herein, the term "complementary determining region" or "CDR" refers to the amino acid residue responsible for antigen binding in the variable region of an antibody. The precise boundaries of these amino acid residues may be defined in accordance with the various numbering systems known in the art, for example as defined in the AbM numbering system (Martin ACR, Cheetham JC, Rees AR (1989) Modelling antibody hypervariable loops: A combined algorithm. Proc Natl Acad Sci USA 86:9268-9272) or the IMGT numbering system (Lefranc et al., Dev. Comparat. Immunol. 27:55-77, 2003). For a given antibody, a person skilled in the art will easily identify the CDRs defined by various numbering systems. And the corresponding relationship between different numbering systems is well known to those skilled in the art (for example, see Lefranc et al., Dev. Comparat. Immunol. 27: 55-77, 2003).

Herein, the CDRs contained in the antibody or the antigen-binding fragment thereof according to the present invention may be determined according to various numbering systems known in the art. In certain embodiments, the CDRs contained in the antibody or the antigen-binding fragment thereof according to the present invention are preferably determined by the IMGT, Kabat, Chothia or AbM numbering system.

The following general rules (available at www.bioinf.org.uk: Professor Andrew C.R. Martin's team) can be used to define CDRs in the sequence of an antibody, which includes amino acids that interact specifically with amino acids composed of antigenic epitopes bound to antibodies. In rare cases, these generally constant features do not appear; but Cys residues are the most conserved feature.

| Ring | Kabat | AbM | Chothia¹ | Chothia² | IMGT |
|---|---|---|---|---|---|
| L1 | L24--L34 | L24--L34 | L24--L34 | L30--L36 | L27--L32 |
| L2 | L50--L56 | L50--L56 | L50--L56 | L46--L55 | L50--L52 |
| L3 | L89--L97 | L89--L97 | L89--L97 | L89--L96 | L89--L97 |
| H1 | H31--H35B (Kabat numbering system)³ | H26-H35B | H26--H32..34 | H30--H35B | H26-H35B |
| H1 | H31--H35 (Chothia numbering system) | H26--H35 | H26--H32 | H30--H35 | H26--H33 |
| H2 | H50--H65 | H50--H58 | H52--H56 | H47--H58 | H51--H56 |
| H3 | H95--H102 | H95-H102 | H95--H102 | H93--H101 | H93--H102 |
| 1 Some of these numbering systems, notably the Chothia numbering system, differ depending on the publications being reviewed. | | | | | |
| 2 Any numbering system other than the Contact numbering system using the definitions of Chothia or Martin (extended Chothia) may be used for the definition of CDRs. | | | | | |
| 3 When numbered using the Kabat numbering system, the end of the Chothia CDR-H1 ring varies between H32 and H34, depending on the ring length. (The reason is that the Kabat numbering system places the insertion points at H35A and H35B). | | | | | |
| If neither H35A nor H35B is present, the end of the ring is located at H32; | | | | | |
| if only H35A is present, the end of the ring is located at H33; | | | | | |
| if both H35A and H35B are present, the end of the ring is located at H34. | | | | | |

The entire amino acid sequence of the V_{H} is usually numbered according to Kabat, while the three CDRs in the variable region can be defined according to any of the numbering systems described above. In certain embodiments, the amino acid sites in V_{H} may be numbered sequentially from amino acid site 1 up to the end of the sequence, or may be numbered according to Kabat. Unless otherwise noted, amino acid sites in V_{H} and V_{L} described herein are numbered and defined sequentially.

The amino acid sites in the heavy chain constant region can be numbered sequentially from amino acid site 1 to the end of the sequence, or can be numbered according to Eu. The amino acid sequence of the heavy chain constant region of IgG1 has 330 amino acids, sequentially numbered from 1 to 330. The corresponding sequence numbered according to Eu begins at site 118 and ends at site 447. Unless otherwise stated, the amino acid sites of the heavy and light chains described herein are sequentially numbered and defined.

As used herein, the term "framework region" or "FR" residues refer to those amino acid residues in the variable region of an antibody other than the CDR residues as defined above.

The term "antibody" is not limited by any particular method for producing antibodies. For example, antibodies include recombinant antibodies, monoclonal antibodies, and polyclonal antibodies. The antibodies may be antibodies of different isotypes, such as IgG (e.g., an IgG1, IgG2, IgG3, or IgG4 subtype), IgA1, IgA2, IgD, IgE, or IgM antibody.

As used herein, the term "antigen-binding fragment" of an antibody refers to a molecule other than an intact antibody, which includes a part of an intact antibody that binds to the antigen to which the intact antibody binds. For example, the antigen-binding fragment may be a polypeptide of a fragment of a full-length antibody that maintains the ability to specifically bind to the same antigen that a full-length antibody binds to, and/or competes with a full-length antibody for specific binding to the antigen and which is also called an "antigen-binding moiety". In general, see Fundamental Immunology, Ch. 7 (Paul, W., ed., 2nd Edition, Raven Press, N.Y. (1989), which is incorporated herein by reference in its entirety for all purposes. Antigen-binding fragments of an antibody can be produced by recombinant DNA technologies or by enzymatic or chemical cleavage of an intact antibody. Non-limiting examples of antigen-binding fragments include Fab, Fab', Fab'-SH, F(ab')₂, Fd, Fv, dAb and complementary determining region (CDR) fragments, single chain antibody (e.g., scFv), chimeric antibody, diabody, linear antibody, nanobody (by technology from Domantis), domain antibody (by technology from Ablynx), and a polypeptide comprising at least a portion of an antibody sufficient to confer the specific antigen-binding ability to the polypeptide. Engineering modified antibody variants are reviewed in Holliger et al., 2005; Nat Biotechnol, 23: 1126-1136.

As used herein, the term "full-length antibody" means an antibody consisting of two "full-length heavy chains" or "heavy chains" and two "full-length light chains" or "light chains". The "full-length heavy chain" or "heavy chain" here refers to a polypeptide chain which, from N-terminal to C-terminal, consists of a heavy chain variable region (VH), a heavy chain constant region CH1 domain, a hinge region (HR), a heavy chain constant region CH2 domain and a heavy chain constant region CH3 domain; and, when the full-length antibody is an IgE isotype, it optionally further comprises a heavy chain constant region CH4 domain. Preferably, a "full-length heavy chain" is a polypeptide chain consisting of VH, CH1, HR, CH2, and CH3 from N-terminal to C-terminal. The "full-length light chain" or "light chain" is a polypeptide chain consisting of a light chain variable region (VL) and a light chain constant region (CL) from N-terminal to C-terminal. Two pairs of full-length antibody chains are linked by a disulfide bridge between CL and CH1 and a disulfide bridge between HRs of two full-length heavy chains. The full-length antibody of the invention may be from a single species, such as human; and can also be a chimeric antibody or a humanized antibody. The full-length antibody of the invention comprises two antigen-binding sites formed by VH and VL pairs, respectively, and the two antigen-binding sites specifically identify/bind to the same antigen.

As used herein, the term "Fd fragment" means an antibody fragment consisting of VH and CH1 domains; the term "dAb fragment" means an antibody fragment consisting of a VH domain (Ward et al., Nature 341: 544 546 (1989)); the term "Fab fragment" means an antibody fragment consisting of VL, VH, CL and CH1 domains; the term "F(ab')2 fragment" means an antibody fragment comprising two Fab fragments linked by a disulfide bridge on the hinge region; the term "Fab' fragment" means a fragment obtained by reducing the disulfide bridge linking two heavy chain fragments in the F(ab')2 fragment and consisting of a complete light chain and a Fd fragment (composed of VH and CH1 domains) of a heavy chain. The term "Fab '-SH" means a Fab fragment containing free sulfhydryl groups.

As used herein, the term "Fv fragment" means an antibody fragment consisting of VL and VH domains of a single-arm of an antibody. The Fv fragment is generally considered to be the smallest antibody fragment that can form a complete antigen-binding site. It is generally thought that six CDRs confer antigen-binding specificity to an antibody. However, even one variable region (such as a Fd fragment, which contains only three antigen-specific CDRs) can identify and bind to antigen, although possibly having a lower affinity than a complete binding site.

As used herein, the term "Fc fragment" means an antibody fragment formed by linking the second and third constant regions of the first heavy chain of an antibody to the second and third constant regions of the second heavy chain of the antibody via a disulfide bridge. The Fc fragment of an antibody has many different functions, but does not involve in antigen binding.

As used herein, the term "scFv" refers to a single polypeptide chain comprising VL and VH domains, wherein the VL and VH are linked by a linker (see, for example, Bird et al., Science 242: 423-426 (1988); Huston et al., Proc. Natl. Acad. Sci. USA 85: 5879-5883 (1988); and Pluckthun, The Pharmacology of Monoclonal Antibodies, vol. 113, Ed. Roseburg and Moore, Springer-Verlag, New York, pp. 269-315 (1994)). Such scFv molecules may have a general structure: NH2-VL-Linker-VH-COOH or NH2-VH-Linker-VL-COOH. A suitable linker in the art consists of repeated GGGGS amino acid sequences or variants thereof. For example, a linker having an amino acid sequence (GGGGS)4 or a variant thereof can be used (Holliger et al. (1993), Proc. Natl. Acad. Sci. USA 90: 6444-6448). Other linkers that may be used in the invention are described in Alfthan et al. (1995), Protein Eng. 8: 725-731; Choi et al. (2001), Eur. J. Immunol. 31: 94-106; Hu et al. (1996), Cancer Res. 56: 3055-3061; Kipriyanov et al. (1999), J. Mol. Biol. 293: 41-56 and Roovers et al. (2001), Cancer Immunol. In some cases, a disulfide bridge may also be present between VH and VL of scFv.

As used herein, the term "diabody" means that the VH and VL domains are expressed on a single polypeptide chain but the used linker is too short to allow for pairing between two domains of the same chain, thus forcing the domain to pair with a complementary domain of the other chain and producing two antigen-binding sites (see, for example, Holliger P. et al., Proc. Natl. Acad. Sci. USA 90: 6444-6448 (1993), and Poljak R. J. et al., Structure 2:1121-1123 (1994)).

Each of the above antibody fragments retains the ability to specifically bind to the same antigen as bound by the full-length antibody, and/or competes with the full-length antibody for specific binding to an antigen. As described herein, an antigen-binding fragment (e.g., the antibody fragment described above) may be obtained from a given antibody (e.g., the antibody of the invention) using conventional technologies known to those skilled in the art, and the antigen-binding fragment of the antibody is specifically screened in the same manner as for an intact antibody.

As used herein, the term "multispecific antibody" refers to antibodies having a variety of different antigen-binding specificities, comprising, for example, bispecific antibodies, tri-specific antibodies, and quad-specific antibodies. "Bispecific antibody" means an antibody having two different antigen-binding specificities, which is a conjugate formed by conjugating a first antibody (or a fragment thereof) to a second antibody (or a fragment thereof) or an antibody analogue via a conjugating arm by means, including but not limited to chemical reactions, gene fusion and enzymatic stimulation. "Multispecific antibodies" include, for example, tri-specific antibodies, which are antibodies having three different antigen-binding specificities, and quad-specific antibodies, which are antibodies having four different antigen-binding specificities.

As used herein, the terms "monoclonal antibody", "McAb" and "mAb" have the same meaning and are used interchangeably, and they refer to an antibody or a fragment of an antibody from a group of highly homologous antibody molecules, that is, a group of identical antibody molecules, excluding natural mutations that may occur spontaneously. A monoclonal antibody has a high specificity to a single epitope on an antigen. Compared to a monoclonal antibody, a polyclonal antibody usually contains at least two or more different antibodies, which usually identify different epitopes on an antigen. In addition, the modifier "monoclonal" simply indicates that the properties of the antibody are obtained from a population of highly homologous antibodies and cannot be understood as requiring any particular method to prepare the antibody.

As used herein, the term "chimeric antibody" means such an antibody where one part of its light chain or/and heavy chain is derived from an antibody (which may be derived from a specific species or belong to a specific antibody class or subclass), and the other part of the light chain or/and heavy chain is derived from another antibody (which may be derived from the same or different species or belong to the same or different antibody class or subclass), which in any case retains binding activity to a target antigen. For example, the term "chimeric antibody" may include an antibody in which the VH and the VL are derived from a first antibody (e.g., human antibody) and the CH and the CL are derived from a second antibody (e.g., murine antibody). For example, antibodies produced by immunizing fully human transgenic mice can be called chimeric antibodies, which consist of a fully human variable region and a murine constant region.

As used herein, the term "humanized antibody" refers to an antibody that can be prepared by replacing part of a human antibody with part of a non-human antibody prepared by immunizing against mammals other than humans. Typically, all or some of the CDRs of a humanized antibody are derived from a non-human antibody (donor antibody), and all or some of the non-CDRs (e.g., FRs in the variable region and/or the constant region) are derived from a human immunoglobulin (receptor antibody). For example, humanized antibodies may be prepared by transplanting CDR sequences from other mammalian germlines into human framework sequences.

As used herein, the term "human antibody" refers to the inclusion of only antibodies that have sequences of immunoglobulins derived from human germlines. It is understood by those skilled in the art that human antibodies do not exclude the inclusion of amino acid residues not encoded by ethnogenic immunoglobulin sequences, such as mutations introduced by random or in vitro site-specific mutations or by in vivo somatic mutations. The human antibody may be prepared by using transgenic mice (XenoMouse (Chemical Biology (2000), vol. 7, issue 8, p.R185-6), HuMAb-Mouse (Infection and Immunity (2002), vol. 70, issue 2, p. 612-9), TC mouse (Biotechnology and Genetics Enginnering Revew (2002), vol. 19, p. 73-82), and KM mouse (Cloning Stem Cells (2002). Vol. 4, issue1, p. 91-102)), where the human immunoglobulin gene is transferred and the target antibody can be mass-produced by isolating antibody-producing lymphocytes from mice to hybridomas. The human antibody may also be prepared by phage display (FEBS Letter (1998), vol. 441, p. 20-24). In this method, by using a phage that integrates the human antibody gene into the circular single-stranded DNA, the human antibody may be expressed on the phage surface in the form of fusion with the phage's coat protein. The human antibody may also be prepared by using transgenic mice with the gene transferred from the variable region of human immunoglobulin. The mice can produce a chimeric antibody consisting of a human variable region and a mouse constant region, and the human variable region can then be linked to a constant region of human immunoglobulin using methods known in the art to produce a human antibody. For example, the DNA encoding the VH is operationally linked to another DNA molecule encoding a CH to obtain a full-length heavy chain gene. The sequence of the human CH gene is known in the art (see e.g. Kabat, E.A. et al. (1991) Sequences of Proteins of Immunological Interest, Fifth Edition, U.S. Department of Health and Human Services, NIH Publication No. 91-3242), and DNA fragments containing these regions may be obtained by standard PCR amplification. The CH may be the constant region of IgG1, IgG2, IgG3, IgG4, IgA, IgE, IgM or IgD, but is usually preferred to the constant region of IgG1 or IgG4. For example, the DNA encoding VL is operationally linked to another DNA molecule encoding the CL to obtain the full-length light chain gene (and the light chain gene of Fab). The sequence of the human CL gene is known in the art (see e.g. Kabat, E.A. et al. (1991) Sequences of Proteins of Immunological Interest, Fifth Edition, U.S. Department of Health and Human Services, NIH Publication No. 91-3242), and DNA fragments containing these regions may be obtained by standard PCR amplification. The CL can be the κ or λ constant region, but the κ constant region is usually preferred.

As used herein, the term "variant", in the context of a polypeptide (including a polypeptide), also refers to a polypeptide or peptide that contains an amino acid sequence that has been altered by the introduction of amino acid residues by substitution, deletion, or addition. In some cases, the term "variant" also refers to a polypeptide or peptide that has been modified (i.e., by covalently linking any type of molecule to a polypeptide or peptide). For example, but unrestrictedly, polypeptides may be modified, for example, by glycosylation, acetylation, pegylation, phosphorylation, amidation, derivatization by known protective/blocking groups, proteolytic cleavage, attachment to cellular ligands or other proteins, etc. Derived polypeptides or peptides may be produced by chemical modifications using techniques known to those skilled in the art, including but not limited to specific chemical cleavage, acetylation, formylation, and metabolic synthesis of tunicamycin. In addition, a variant has similar, identical, or improved functions to the polypeptide or peptide from which the variant is derived.

As used herein, the term "specifically binding" refers to non-random binding reaction between two molecules, such as reaction between an antibody and an antigen against which the antibody is. The intensity or affinity of the specific binding interaction can be indicated by the dissociation equilibrium constant (KD) of the interaction or the median effective concentration (EC₅₀).

The specific binding properties between two molecules can be assayed using methods well known in the art. One of the methods involves measuring the rate of formation and dissociation of antigen-binding site/antigen complexes. Both "association rate constant" (Kₐ or Kon) and "dissociation rate constant" (Kdis or Koff) can be calculated from the concentration and the actual rates of association and dissociation (see Malmqvist M, Nature, 1993, 361: 186-187). The ratio of Kdis/Kon is equal to the dissociation constant KD (see Davies et al., Annual Rev Biochem, 1990; 59: 439-473). The values of KD, Kon and Kdis can be measured by any effective method. In certain embodiments, the dissociation constant can be measured by bioluminescence interferometry (e.g., ForteBio Octet assay). In addition, the dissociation constant can also be measured by surface plasmon resonance technology (such as Biacore) or Kinexa.

As used herein, the term "vector" refers to a nucleic acid vehicle into which a polynucleotide can be inserted. When enabling the expression of a protein encoded by the inserted polynucleotide, the vector is named expression vector. Vectors can be introduced into host cells through transformation, transduction, or transfection, so that the carried genetic material elements can be expressed in host cells. Vectors are well known to those skilled in the art, including but not limited to: plasmids; phagemids; cosmids; artificial chromosomes, such as yeast artificial chromosomes (YAC), bacterial artificial chromosomes (BAC), or P1-derived artificial chromosomes (PAC); phages, such as λ phage or M13 phage, and animal viruses. Animal viruses that can be used as vectors include, but not limited to, retroviruses (including lentivirus), adenoviruses, adeno-associated viruses, herpesviruses (e.g., herpes simplex virus), poxviruses, baculoviruses, papillomaviruses, papovaviruses (e.g., SV40). A vector may contain various elements controlling expression, including but not limited to a promoter sequence, a transcription initiation sequence, an enhancer sequence, a selection element, and a reporter gene. In addition, the vector may also contain an origin of replication.

Expression and cloning vectors contain nucleic acid sequences that enable the vectors to replicate in one or more selected host cells. Typically, in a clonal vector, this sequence enables the vector to replicate independently of the chromosome DNA of a host and includes a replication origin or an autonomously replicated sequence. The term "expression vector" as used herein refers to a vector containing a recombinant polynucleotide, which comprises an expression regulatory sequence that is effectively linked to the nucleotide sequence to be expressed. The expression vector comprises sufficient cis-acting elements for expression. Additional elements for expression may be provided by host cells or in vitro expression systems. Expression vectors include all those known in the art, such as cosmids, plasmids (e.g., naked or contained in liposomes) and viruses (such as lentiviruses, retroviruses, adenoviruses and adeno-associated viruses).

As used herein, the term "host cell" refers to a cell into which a vector can be introduced, including, but not limited to, prokaryotic cells such as E. coli or Bacillus subtilis, fungal cells such as yeast cells or Aspergillus, insect cells such as S2 drosophila cell or Sf9, or animal cells such as fibroblasts, NS0 cells, Vero cells, Hela cells, COS cells, CHO cells (e.g., CHO-K1, CHO-S, CHO DXB11, ExpiCHO, CHO DG44, or CHO-EBNA cells), ExpiCHO cells, HEK293 cells, Expi293 cells, BHK cells, and MDCKII cells.

As used herein, the term "identity" refers to the sequence matching degree between two polypeptides or two nucleic acids. When a position in each of two sequences for comparison is occupied by the same base or amino acid monomer subunit (for example, a position in each of two DNA molecules is occupied by adenine, or a position in each of two polypeptides is occupied by lysine), then the molecules are identical at that position. "% identity" between two sequences is a function that the number of matching positions shared by the two sequences ÷ the number of positions to be compared × 100. For example, if six of ten positions in two sequences match, then the two sequences are 60% identical. For example, the DNA sequences CTGACT and CAGGTT have 50% identity (three of the total six positions match). Typically, comparison is made when two sequences are aligned to produce maximum identity. Such an alignment can be realized by using, for example, the method of Needleman et al. (1970) J. Mol. Biol. 48: 443-453, which can be conveniently performed by a computer program such as Align program (DNAstar, Inc). The % identity between two amino acid sequences can be determined by the algorithm of E. Meyers and W. Miller (Comput. Appl Biosci., 4: 11-17 (1988)) incorporated in the ALIGN program (version 2.0), using the PAM120 weight residue table, a gap length penalty of 12 and a gap penalty of 4. Additionally, the % identity between two amino acid sequences can be determined by the algorithm of Needleman and Wunsch (J Mol Biol. 48: 444-453 (1970)) in the GAP program incorporated in the GCG software package (available at www.gcg.com), using either a Blossum Matrix 62 or PAM250, and a gap weight of 16, 14, 12, 10, 8, 6 or 4 and a length weight of 1, 2, 3, 4, 5 or 6.

As used herein, the term "conservative substitution" means an amino acid substitution that does not adversely affect or alter the expected properties of a protein/polypeptide comprising an amino acid sequence. For example, a conservative substitution may be introduced by standard technologies known in the art, such as site-directed mutagenesis and PCR-mediated mutagenesis. A conservative amino acid substitution includes a substitution of an amino acid residue with an amino acid residue having a similar side chain, e.g., with a residue that is physically or functionally similar to the corresponding amino acid residue (e.g., having similar size, shape, charge, chemical property, including the ability to form covalent or hydrogen bonds, etc.). Families of amino acid residues having similar side chains have been defined in the art. These families include amino acids having alkaline side chains (e.g., lysine, arginine and histidine), acidic side chains (e.g. aspartic acid, glutamic acid), uncharged polar side chains (e.g. glycine, asparagine, glutamine, serine, threonine, tyrosine, cysteine, tryptophan), nonpolar side chains (e.g. alanine, valine, leucine, isoleucine, proline, phenylalanine, methionine), beta branched side chains (e.g., threonine, valine, isoleucine) and aromatic side chains (e.g. tyrosine, phenylalanine, tryptophan, histidine). Therefore, it is preferred to replace a corresponding amino acid residue with another amino acid residue from the same side chain family. Methods for identifying conservative substitution of amino acids are well known in the art (see, for example, Brummell et al., Biochem. 32: 1180-1187 (1993); Kobayashi et al., Protein Eng. 12 (10): 879-884 (1999); and Burks et al., Proc. Natl Acad. Set USA 94: 412-417 (1997), which are incorporated herein by reference).

The compilation of twenty conventional amino acids involved herein follows the conventional usage. See, for example, Immunology-A Synthesis (2nd Edition, E. S. Golub and D. R. Gren, Eds., Sinauer Associates, Sunderland, Mass. (1991)), which is incorporated herein by reference. Herein, the terms "polypeptide" and "protein" have the same meaning and are used interchangeably. Also, in the invention, amino acids are generally expressed by single-letter and three-letter abbreviations well known in the art. For example, alanine may be expressed as A or Ala.

As used herein, the term "pharmaceutically acceptable carrier and/or excipient" means a carrier and/or excipient that is pharmacologically and/or physiologically compatible with the subject and active ingredient, which are well known in the art (see, for example, Remington's Pharmaceutical Sciences. Edited by Gennaro AR, 19th ed. Pennsylvania: Mack Publishing Company, 1995) and include, but not limited to, pH modulators, surfactants, adjuvants, ionic strength enhancers, diluents, osmotic pressure maintaining reagents, delayed absorption reagents, preservatives. For example, the pH modulators include, but not limited to, phosphate buffers. The surfactants include, but not limited to cationic, anionic, or nonionic surfactants, such as Tween-80. The ionic strength enhancers include, but not limited to, sodium chloride. The preservatives include, but not limited to, various antibacterial and antifungal agents, such as p-hydroxybenzoate, trichloro-tert-butyl alcohol, phenol, sorbic acid, etc. The osmotic pressure maintaining reagents include, but not limited to, sugars, NaCl and the like. The delayed absorption reagents include, but not limited to, monostearates and gelatin. Diluents include, but not limited to, water, aqueous buffers (e.g., buffer saline), alcohols and polyols (e.g., glycerol), etc. The preservatives include, but not limited to, various antibacterial and antifungal agents, such as thiomersalate, 2-phenoxyethanol, p-hydroxybenzoate, trichloro-tert-butyl alcohol, phenol, sorbic acid, etc. The stabilizers have a meaning commonly understood by those skilled in the art, which can stabilize the desired activity of the active ingredient in drug, including but limited to, sodium glutamate, gelatin, SPGA, sugars (such as sorbitol, mannitol, starch, sucrose, lactose, dextran, or glucose), amino acids (such as glutamic acid, glycine), proteins (such as dried whey, albumin, or casein) or degradation products thereof (such as lactalbumin hydrolysate).

As used herein, the term "prevention" refers to a process for preventing or delaying the onset of a disease or condition or symptom (e.g., a tumor) in vivo in a subject. As used herein, the term "treatment" refers to a process for achieving a beneficial or desired clinical result. For purposes of the invention, beneficial or desired clinical result includes, but not limited to, alleviation of a symptom, diminishment of extent of disease, stabilizing (i.e., not worsening) state of a disease, delay or slowing of disease progression, amelioration or palliation of disease state, and remission (whether partial or all) of symptoms, no matter detectable or undetectable. In addition, "treatment" can also mean prolonging survival as compared to the expected survival (if without treatment).

As used herein, the term "subject" refers to a mammal, such as a primate mammal, such as a human. In some embodiments, the subject (e.g., human) has a tumor, or is at a risk of suffering from such disease.

As used herein, the term "effective amount" refers to an amount sufficient to achieve or at least partially achieve a desired effect. For example, an effective amount for preventing a disease is an amount sufficient to prevent, stop, or delay the onset of the disease (e.g., a tumor); a therapeutic effective amount refers to an amount sufficient to cure or at least partially prevent a disease and its complications of a patient who have already suffered from the disease. To determine such effective amount is within the scope of capability of those skilled in the art. For example, the amount effective for a therapeutic use will depend on the severity of the disease to be treated, the general state of the patient's own immune system, the general condition of the patient such as age, weight and gender, route of administration, and other treatments administered concurrently, etc.

As used herein, the term "effector function" refers to those biological activities which contribute to the Fc region of an antibody (Fc region of a natural sequence or Fc region of an amino acid sequence variant) and vary with antibody isotypes. Examples of effector functions of antibodies include, but are not limited to: Fc receptor binding affinity, antibody-dependent cell-mediated cytotoxicity (ADCC), complement-dependent cytotoxicity (CDC), antibody-dependent cellular phagocytosis (ADCP), downregulation of cell surface receptors (e.g., B-cell receptors), B-cell activation, cytokine secretion, and half-life/clearance of antibodies and antigen-antibody complexes. Methods of altering the effector function of antibodies are known in the art, for example, by introducing mutations in the Fc region.

As used herein, the term "antibody-dependent cell-mediated cytotoxicity (ADCC)" refers to a form of cytotoxicity in which Ig binds to an Fc receptor (FcR) present on cytotoxic cells, such as natural killer (NK) cells, neutrophils, or macrophages, such that these cytotoxic effector cells specifically bind to the target cell to which the antigen is attached, and then kill the target cell by secreting cytotoxins.

As used herein, the term "complement-dependent cytotoxicity" refers to the cytotoxic effects in which the complement participates, that is, by binding specific antibodies to the corresponding antigens on the surface of the cell membrane, a complex is formed to activate the classical complement pathway, and the resulting membrane attack complex plays a lytic effect on the target cells.

As used herein, the term "antibody-mediated endocytosis" refers to the phenomenon of an antibody crossing the cell membrane after binding to a cell surface antigen. Endocytosis includes antibody-mediated endocytosis of receptors such as HER3.

Herein, combination therapy includes the combinations of an anti-HER3 antibody or its antigen-binding fragment covered by the present invention with one or more additional active therapeutic agents (e.g., a chemotherapeutic agent) for secondary therapy or other preventive or therapeutic modes (e.g., radiotherapy).

In such combination therapies, the various active agents often have different complementary mechanisms of action, and the combination therapies may lead to synergistic effects. Combination therapies include therapeutic agents that affect the immune response (e.g., enhancing or activating the response) and therapeutic agents that affect (e.g. inhibiting or killing) tumor/cancer cells. Combination therapies can reduce the likelihood of drug-resistant cancer cells developing. Combination therapies may allow a dose reduction of one or more of the reagents to reduce or eliminate adverse effects associated with one or more of the reagents. Such combination therapies may have a synergistic therapeutic or preventive effect on an underlying disease, disorder or symptom.

In this context, "combination" includes therapies that can be administered separately, such as separately formulated for individual administration (for example, may be provided in a set), and therapies that can be administered together as a single formulation (i.e., "co-formulation"). In some embodiments, the anti-B7-H3 antibody or the antigen-binding fragment thereof according to the present invention may be administered sequentially. In other embodiments, the anti-B7-H3 antibody or the antigen-binding fragment thereof may be administered simultaneously. The antibody or the antigen-binding fragment thereof according to the present invention may be used in any manner in combination with at least one other (active) agent.

Herein, B7-H3 positive was obtained by immunohistochemistry and staining intensity evaluation by professional clinicopathologists.

The terms "cancer" and "tumor", used interchangeably, refer to a large group of diseases characterized by the uncontrolled growth of abnormal cells in the body. Unregulated cell division may lead to the formation of malignant tumors or cells that invade neighboring tissues and may metastasize to distant parts of the body through the lymphatic system or bloodstream. Cancers include benign and malignant cancers as well as dormant tumors or micrometastases. Cancer also includes hematologic malignancies.

The term "hematologic malignancies" includes lymphoma, leukemia, myeloma, or lymphoid malignancies, as well as splenic and lymph node tumors. For example, lymphomas include B-cell lymphomas and T-cell lymphomas. B-cell lymphomas include, for example, Hodgkin's lymphoma. T-cell lymphomas, include, for example, cutaneous T-cell lymphoma. Hematological malignancies also include leukemia, such as secondary leukemia or acute lymphoblastic leukemia. Hematologic malignancies also include myeloma (e.g., multiple myeloma) and other hematological and/or B-cell or T-cell related cancers.

The embodiments of the present invention are described in detail below in conjunction with the drawings and examples, but those skilled in the art will understand that the following drawings and examples are used only to illustrate the invention and not to limit the scope of the invention. The objectives and advantages of the invention will become implementable for those skilled in the art according to the accompanying drawings and the following detailed description of the preferred embodiments.

### BRIEF DESCRIPTION OF THE DRAWINGS

FIG. 1A: Detection of binding affinity of anti-human-B7-H3 human antibody 2# to human B7-H3-4Ig protein
FIG. 1B: Detection of binding affinity of anti-human-B7-H3 human antibody 2# to monkey B7-H3-4Ig protein
FIG. 2: Detection of binding affinity of anti-human-B7-H3 human antibody to human B7-H3-4Ig protein
FIG. 3A: Detection of binding affinity of anti-human-B7-H3 human antibody to human colon cancer cell HT29
FIG. 3B: Detection of binding affinity of anti-human-B7-H3 human antibody to human gastric cancer cell NCI-N87
FIG. 3C: Detection of binding affinity of anti-human-B7-H3 human antibody to human mammary squamous cell HCC1806
FIG. 3D: Detection of binding affinity of anti-human-B7-H3 human antibody to human non-small cell lung cancer cell HCC827
FIG. 3E: Detection of binding affinity of anti-human-B7-H3 human antibody to CHO-S-human B7-H3-4Ig
FIG. 3F: Detection of binding affinity of anti-human-B7-H3 human antibody to CHO-S-human B7-H3-2Ig
FIG. 4A: Detection of binding affinity of anti-human-B7-H3 human antibody to CHO-S-monkey B7-H3
FIG. 4B: Detection of binding affinity of anti-human-B7-H3 human antibody to CHO-S-rat B7-H3
FIG. 5: Detection of ADCC of anti-human-B7-H3 human antibody
FIG. 6: Detection of CDC of anti-human-B7-H3 human antibody
FIG. 7A: Detection of endocytosis of anti-human-B7-H3 human antibody to human gastric cancer cell NCI-N87
FIG. 7B: Detection of endocytosis of anti-human-B7-H3 human antibody to human mammary squamous cell HCC1806
FIG. 7C: Detection of endocytosis of anti-human-B7-H3 human antibody to human non-small cell lung cancer cell HCC827
FIG. 8: Detection of epitope competition of anti-human-B7-H3 human antibody
FIG. 9: Detection of specificity of anti-human-B7-H3 human antibody

### Information of sequences

Information of the sequences to which the invention relates is shown in the table below:

| SEQ ID NO: | Description | |
|---|---|---|
| 1 | Amino acid sequence of VH in 20G11G6/2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSS |
| 2 | Amino acid sequence of VL in 20G11G6/2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIK |
| 3 | Amino acid sequence of VH in 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSS |
| 4 | Amino acid sequence of VL in 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIK |
| 5 | CDR-H1 of 20G11G6/2# (IMGT) | GGSNSSSNW |
| 6 | CDR-H2 of 20G11G6/2# (IMGT) | ISPTGFT |
| 7 | CDR-H3 of 20G11G6/2# (IMGT) | ARDRLYFDF |
| 8 | CDR-L1 of 20G11G6/2#/2#8890 (IMGT) | QSVSSN |
| 9 | CDR-L2 of 20G11G6/2#/2#8890 (IMGT) | GAS |
| 10 | CDR-L3 of 20G11G6/2#/2#8890 (IMGT/Chothia/Kabat/ AbM) | QQYNNWPIT |
| 11 | CDR-H1 of 20G11G6/2# (Chothia) | GGSNSSSN |
| 12 | CDR-H2 of 20G11G6/2# (Chothia) | SPTGF |
| 13 | CDR-H3 of 20G11G6/2# (Chothia/Kabat/AbM) | DRLYFDF |
| 14 | CDR-L1 of 20G11G6/2#/2#8890 (Chothia/Kabat/AbM) | RASQSVSSNLA |
| 15 | CDR-L2 of 20G11G6/2#/2#8890 (Chothia/Kabat/AbM) | GASTRAT |
| 16 | CDR-H1 of 20G11G6/2# (Kabat) | SSNWWT |
| 17 | CDR-H2 of 20G11G6/2# (Kabat) | EISPTGFTSYSPSLKS |
| 18 | CDR-H1 of 2#8890 (IMGT) | GGSDSSTNW |
| 19 | CDR-H2 of 2#8890 (IMGT) | ISPIGFT |
| 20 | CDR-H3 of 2#8890 (IMGT) | ARDRLYFAF |
| 21 | CDR-H1 of 2#8890 (Chothia) | GGSDSSTN |
| 22 | CDR-H2 of 2#8890 (Chothia) | SPIGF |
| 23 | CDR-H3 of 2#8890 (Chothia/ Kabat/AbM) | DRLYFAF |
| 24 | CDR-H1 of 2#8890 (Kabat) | STNWWT |
| 25 | CDR-H2 of 2#8890 (Kabat) | EISPIGFTSYSPSLRS |
| 26 | CDR-H1 of 20G11G6/2# (AbM) | GGSNSSSNWWT |
| 27 | CDR-H2 of 20G11G6/2# (AbM) | EISPTGFTS |
| 28 | CDR-H1 of 2#8890 (AbM) | GGSDSSTNWWT |
| 29 | CDR-H2 of 2#8890 (AbM) | EISPIGFTS |
| 30 | Amino acid sequence of CH of IgG1 | |
| 31 | Amino acid sequence of mutated CH of IgG1 | ASTKGPSVFPLAPSSKSTSGGTAALGCLVKDYFPEPVT VSWNSGALTSGVHTFPAVLQSSGLYSLSSVVTVPSSSL GTQTYICNVNHKPSNTKVDKKVEPKSCDKTHTCPPCP APEAAGAPSVFLFPPKPKDTLMISRTPEVTCVVVDVSH EDPEVKFNWYVDGVEVHNAKTKPREEQYNSTYRVVS VLTVLHQDWLNGKEYKCKVSNKALPAPIEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| 32 | Amino acid sequence of human light chain κ constant region | |
| 33 | Nucleotide sequence of VH of 20G11G6/2# | CAGGTTCAATTGCAAGAAAGCGGCCCCGGGCTGGTA AAACCAAGTGGCACCCTGAGCCTGACGTGTGCTGTG TCTGGGGGGAGCAATTCCTCAAGCAATTGGTGGACA TGGGTGAGGCAGTCCCCCGGAAAGGGCTTGGAATG GATCGGCGAGATCAGCCCTACAGGTTTTACCAGCTA CTCCCCTAGCCTGAAATCCAGGGTGACGATCAGTGT CGACAAGTCTAAAACACAGTTCTCTCTGAACCTTTC AAGTGTCACTGCAGCCGATACTGCTGTGTATTACTGC GCCAGAGATCGGCTGTATTTTGACTTTTGGGGCCAG GGCACTCTGGTCACAGTGTCTAGC |
| 34 | Nucleotide sequence of VL of 20G1 1G6/2# | GAAATAGTGATGACCCAATCCCCCGCAACTCTGTCT GTGTCCCCTGGGAAGAGAGCCACCCTTTCCTGTCGC GCCTCACAATCCGTGAGCTCAAATCTGGCATGGTATC AACAGAAACCAGGCCAAGCGCCTCGGCTGCTGATTT ACGGCGCCTCTACCCGCGCTACCGGAATCCCAGCAC GCTTTAGCGGCTCAGGGTCCGGCACTGAGTTCACCT TGACAATTAGTAGTCTGCAGTCCGAAGATTTTGCCGT CTATTACTGCCAGCAGTATAATAACTGGCCAATCACC TTTGGCCAGGGCACACGCCTGGAGATCAAG |
| 35 | Nucleotide sequence of VH of 2#8890 | CAAGTACAACTCCAAGAAAGTGGGCCAGGACTCGT GCAACCATCTGGCACCCTCTCTCTCACCTGCGCAGT GTCTGGCGGCTCCGATTCTTCCACCAACTGGTGGAC CTGGGTGAGACAGTCCCCAGGCCAAGGATTGGAATG GATTGGCGAGATTTCCCCTATTGGGTTCACCAGCTAC AGCCCCAGCCTGAGGAGCAGGGTGACTATCAGCGTG GATAAGTCTAAAACCCAGTTCAGTCTCAAACTGAGT AGTGTGACTGCCGCGGACACGGCAGTGTATTATTGC GCCCGGGACCGCCTTTATTTCGCCTTCTGGGGGCAG GGCACTTTGGTGACCGTCAGTAGC |
| 36 | Nucleotide sequence of VL of 2#8890 | GAGATTGTCATGACACAGAGTCCTGCTACCTTGTCA GTTAGTCCTGGGGAGAGAGCAACACTGTCCTGCCGC GCTAGCCAAAGTGTTAGCTCCAACCTGGCCTGGTAC CAGCAGAAACCCGGGCAGGCACCCAGACTCCTGAT TTACGGAGCCTCTACACGCGCAACTGGAATCCCTGA CCGGTTCTCAGGATCCGGGAGTGGCACGGAATTCAC CTTGACCATTAGCAGCCTGGAATCCGAAGATTTTGCA GTGTATTACTGTCAGCAGTACAACAACTGGCCTATCA CGTTCGGGCAGGGGACCAGATTGGAAATCAAG |
| 37 | Nucleotide sequence of CH of IgG1 | GCATCTACTAAAGGCCCAAGTGTTTTTCCTCTGGCCC CATCCTCAAAAAGCACCTCCGGAGGCACAGCTGCGC TGGGATGCCTCGTGAAAGATTATTTCCCTGAACCTGT TACCGTGTCATGGAACAGCGGCGCTCTCACTTCTGG CGTGCACACATTTCCCGCTGTGTTGCAGTCCAGCGG ACTCTATAGCCTGAGCTCCGTGGTTACAGTGCCCTCT TCATCTTTGGGCACCCAGACCTACATATGCAATGTGA ATCATAAACCGAGCAATACCAAGGTAGATAAAAAGG TAGAGCCAAAAAGTTGCGATAAGACTCATACATGCC CACCTTGTCCTGCCCCGGAGCTGCTCGGAGGACCAA GCGTGTTTCTCTTCCCCCCCAAGCCAAAGGATACCTT GATGATTAGTCGCACCCCAGAAGTGACATGCGTGGT GGTTGACGTCAGCCACGAGGACCCAGAGGTGAAGT TTAATTGGTACGTGGACGGTGTGGAAGTCCACAATG CAAAAACCAAGCCTCGCGAAGAACAGTATAACAGT ACCTACAGGGTCGTCAGTGTCCTGACCGTGCTGCAC CAGGACTGGCTCAATGGAAAGGAATACAAGTGTAAG GTCTCCAACAAGGCCTTGCCAGCTCCCATTGAAAAG ACCATTAGCAAGGCCAAAGGTCAGCCTCGCGAACCA CAAGTGTACACACTGCCTCCGTCACGCGATGAACTT ACCAAAAATCAGGTATCACTGACCTGCCTTGTAAAG GGCTTTTACCCGAGTGACATAGCAGTCGAGTGGGAA TCCAACGGTCAGCCGGAGAACAACTATAAAACCACT CCCCCAGTGCTTGATTCCGATGGCAGCTTCTTCCTGT ACAGTAAACTGACTGTGGATAAGTCTCGCTGGCAGC AGGGCAATGTGTTTTCTTGCTCTGTCATGCACGAGG CACTTCACAACCACTACACACAGAAAAGTCTCAGTC TGAGTCCAGGGAAA |
| 38 | Nucleotide sequence of human light chain κ constant region | AGAACAGTGGCCGCACCAAGCGTGTTTATCTTTCCT CCCTCTGACGAACAATTGAAGAGTGGGACCGCCTCA GTGGTGTGTCTGCTGAACAATTTCTACCCCCGGGAG GCAAAGGTGCAGTGGAAGGTGGATAATGCACTCCAG TCCGGGAATAGTCAGGAAAGCGTGACTGAGCAAGA CAGCAAAGACTCTACATATTCTCTGTCCAGTACCCTG ACCCTCAGCAAGGCTGATTATGAGAAGCACAAGGTG TACGCCTGTGAGGTCACTCACCAGGGACTGAGTTCA CCGGTCACTAAAAGCTTTAACAGAGGAGAGTGC |
| 39 | Nucleotide sequence of mutated CH of IgG1 | GCCTCTACAAAAGGACCATCAGTCTTTCCATTGGCTC CGAGTAGCAAAAGCACCAGCGGTGGAACAGCTGCT CTGGGGTGCCTGGTCAAGGACTATTTCCCCGAGCCT GTGACCGTCTCTTGGAACAGCGGGGCACTGACCTCC GGTGTCCATACATTTCCTGCAGTGCTGCAAAGTTCTG GATTGTATTCACTCTCCTCTGTCGTGACGGTTCCTAG TTCATCCCTGGGCACCCAGACTTATATTTGCAATGTC AACCACAAACCCAGCAATACTAAAGTGGATAAGAAG GTCGAACCCAAGTCTTGCGATAAGACACACACATGC CCTCCTTGTCCCGCACCAGAGGCTGCTGGAGCACCA TCAGTGTTTCTTTTCCCACCTAAGCCCAAGGATACAC TGATGATTAGTCGCACACCCGAAGTGACATGCGTCG TCGTAGACGTGAGCCACGAGGACCCTGAGGTGAAAT TTAACTGGTACGTGGATGGAGTGGAGGTACATAATG CAAAGACAAAACCCCGAGAAGAGCAGTACAACAGC ACTTACAGGGTGGTGTCCGTTCTGACAGTCCTGCAT CAGGATTGGCTTAACGGTAAAGAATATAAGTGTAAA GTTAGTAATAAGGCCCTGCCCGCCCCCATTGAGAAG ACTATTAGTAAGGCCAAGGGACAGCCCAGGGAACCT CAGGTGTATACCCTGCCACCCTCCCGGGACGAACTG ACAAAAAATCAGGTATCATTGACCTGCCTCGTGAAG GGCTTTTATCCCTCAGATATAGCCGTGGAATGGGAGT CAAATGGCCAGCCTGAGAACAATTACAAGACTACGC CTCCCGTGCTGGATTCTGATGGATCATTTTTTCTGTAC AGCAAGCTGACCGTGGATAAGTCCCGGTGGCAGCA GGGGAACGTGTTCTCTTGCTCCGTGATGCACGAGGC ACTTCATAACCATTATACCCAAAAGTCTCTCTCACTG TCCCCCGGCAAA |
| 40 | Amino acid sequence of heavy chain of 2# | QVQLQESGPGLVKPSGTLSLTCAVSGGSNSSSNWWT WVRQSPGKGLEWIGEISPTGFTSYSPSLKSRVTISVDKS KTQFSLNLSSVTAADTAVYYCARDRLYFDFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPELLGGPSVFLFPPKPKDTLMISRTPEVTCVVV DVSHEDPEVKFNWYVDGVEVHNAKTKPREEQYNSTY RVVSVLTVLHQDWLNGKEYKCKVSNKALPAPIEKTIS KAKGQPREPQVYTLPPSRDELTKNQVSLTCLVKGFYP SDIAVEWESNGQPENNYKTTPPVLDSDGSFFLYSKLTV DKSRWQQGNVFSCSVMHEALHNHYTQKSLSLSPGK |
| 41 | Amino acid sequence of light chain of 2# | EIVMTQSPATLSVSPGKRATLSCRASQSVSSNLAWYQ QKPGQAPRLLIYGASTRATGIPARFSGSGSGTEFTLTIS SLQSEDFAVYYCQQYNNWPITFGQGTRLEIKRTVAAP SVFIFPPSDEQLKSGTASVVCLLNNFYPREAKVQWKV DNALQSGNSQESVTEQDSKDSTYSLSSTLTLSKADYE KHKVYACEVTHQGLSSPVTKSFNRGEC |
| 42 | Amino acid sequence of heavy chain of 2#8890 | QVQLQESGPGLVQPSGTLSLTCAVSGGSDSSTNWWT WVRQSPGQGLEWIGEISPIGFTSYSPSLRSRVTISVDKS KTQFSLKLSSVTAADTAVYYCARDRLYFAFWGQGTL VTVSSASTKGPSVFPLAPSSKSTSGGTAALGCLVKDYF PEPVTVSWNSGALTSGVHTFPAVLQSSGLYSLSSVVTV PSSSLGTQTYICNVNHKPSNTKVDKKVEPKSCDKTHT CPPCPAPEAAGAPSVFLFPPKPKDTLMISRTPEVTCVV VDVSHEDPEVKFNWYVDGVEVHNAKTKPREEQYNST YRVVSVLTVLHQDWLNGKEYKCKVSNKALPAPIEKTI SKAKGQPREPQVYTLPPSRDELTKNQVSLTCLVKGFY PSDIAVEWESNGQPENNYKTTPPVLDSDGSFFLYSKLT VDKSRWQQGNVFSCSVMHEALHNHYTQKSLSLSPGK |
| 43 | Amino acid sequence of light chain of 2#8890 | EIVMTQSPATLSVSPGERATLSCRASQSVSSNLAWYQ QKPGQAPRLLIYGASTRATGIPDRFSGSGSGTEFTLTIS SLESEDFAVYYCQQYNNWPITFGQGTRLEIKRTVAAPS VFIFPPSDEQLKSGTASVVCLLNNFYPREAKVQWKVD NALQSGNSQESVTEQDSKDSTYSLSSTLTLSKADYEK HKVYACEVTHQGLSSPVTKSFNRGEC |

### DETAILED DESCRIPTION OF THE EMBODIMENTS

The invention is now illustrated with reference to the following examples which are intended to exemplify but not limit the invention.

Unless indicated otherwise, the processes of molecular biology experiments and immunoassays used in the invention are essentially carried out with reference to the processes described in J. Sambrook et al., Molecular Cloning: A Laboratory Manual, 2nd Ed., Cold Spring Harbor Press, 1989; and F. M. Ausubel et al., Short Protocols in Molecular Biology, 3rd Ed., John Wiley & Sons, Inc., 1995. Those skilled in the art will appreciate that the examples are intended to exemplify the invention and are not intended to limit the scope of the invention.

### Example 1: Preparation of B7-H3 antigen and control antibody protein

### 1.1 Preparation of B7-H3 antigen

The full-length sequences of human B7-H3-2Ig (NCBI: NP_001316557.1), human B7-H3-4Ig (Uniprot: Q5ZPR3), monkey B7-H3-4Ig (XP_015308534.1) and rat B7-H3 (Uniprot: Q7TPB4) were synthesized and constructed on a pLVX vector by GenScript. After plasmid extraction, viruses were packaged and used to construct overexpressed cell lines. Extracellular nucleic acid sequences of human 4Ig and 2Ig, monkey antigen and rat 2Ig were constructed on a pTT5 vector, six histidine tags were introduced into the C-terminal of extracellular segment, and the recombinant plasmid was extracted and then transiently transfected into HEK293-EBNA cells for transient expression after the sequencing, and the cell supernatant was collected 6 days later and purified to obtain human B7-H3-4Ig-His and B7-H3-2Ig-His proteins and monkey B7-H3-4Ig-His protein, and rat B7-H3-2Ig-His protein.

### 1.2 Expression of control B7-H3 antibody

The control B7-H3 antibody was obtained according to patent CN 103687945A. After codon optimization by GenScript, the nucleotide sequences of heavy and light chains in the antibody were synthesized and cloned into the pTT5 vector respectively. After plasmid extraction, the pTT5 plasmids corresponding to the nucleotides of heavy and light chains in the antibody were simultaneously transfected into CHO-S cells, and the cell supernatant was collected by centrifugation and purified using Protein A (MabSelect SuRe, GE) to obtain control antibody protein DS7300.

### Example 2: Preparation of anti-human-B7-H3 chimeric antibody

### 2.1 Mouse immunization

Human transgenic mice H2L2 (Harbour BioMed) (the antibody produced by the mice was a chimeric antibody composed of a variable region of a human antibody and a constant region of rat origin) were immunized using human B7-H3-4Ig-His prepared in 1.1. Immunization was carried out on foot pad, subcutaneous, tail root, and abdominal with Freund's complete adjuvant and Adjuvant system (Sigma); or foot pad immunization with Titermax Gold Adjuvant (Sigma) and Imject Alum (Thermo) was carried out every two to three days. During immunization, the serum titer of the B7-H3 antibody was determined by ELISA every two weeks, and a mouse hybridoma with the best titer was generated according to the solution described in 2.2 below.

### 2.2 Hybridoma fusion screening

1 µg/ml human B7-H3-4Ig-His protein was added to ELISA plates (BIOFIL), with 100 µl/well and incubated at 4 °C overnight. The plates were washed once with 300 µl of PBST (0.2% Tween-20), 100 µl of 2% BSA in PBS was added to each well, followed by incubation at 37 °C for 1 h. 20 µl of hybridoma supernatant was directly added to the plates, followed by incubation at 37°C for 2 h. The solution was then discarded and the plates were washed with PBST (0.2% Tween-20), 320 µl/well, on a washing device for 3 times. The plates were dried and 100 µl of HRP conjugated Goat anti-Rat IgG (Thermo Fisher) diluted with PBST diluted (1:5000) was added to each well, followed by incubation at 37°C for 1 h. The solution was then discarded and the plates were washed with PBST (0.2% Tween-20), 320 µl/well, on a washing device for 5 times. The plates were dried and 100 µl of TMB (Huzhou InnoRagents Co., LTD.) was added to each well for color development in dark, and then 50 µl of 2M H₂SO₄ was added to stop the color development reaction. OD 450 nm was given by a microplate reader. The clones with OD 450 nm 5 times greater than the negative control were identified as positive clones, and the positive clones were then subjected to cross-ELISA with monkey B7-H3-4Ig-His protein (see 2.1), and detected for binding to human breast cancer cell MCF-7, and the best clones were selected for subcloning. Subclones were screened by a similar method, and the best subclones were selected to purify chimeric antibodies.

### Example 3: Evaluation of anti-human-B7-H3 chimeric antibody

### Example 3.1: Evaluation on binding affinity of anti-human-B7-H3 chimeric antibody

All the selected monoclones were transferred to T25 flasks for serum-free culture. 4-6 ml of culture supernatant was affinity purified with Protein-A beads. The protein-A beads-bound antibody protein was eluted with 1M Glycine solution (pH 2.5), and then the antibody protein was neutralized with 1M Tris solution, and the concentration of the antibody protein was quantified with Nanodrop for candidate evaluation.

ELISA for the affinity of B7-H3 chimeric antibody: Human and monkey B7-H3-4Ig-His proteins were diluted with CBS coating solution to 1 µg/ml, then added to the plates with 100 ng/well, and incubated overnight at 4°C. Next day, the ELISA plates were washed once with PBS. Then, 100 µl of 2% BSA was added to each well, followed by incubation at 37°C for 2 h. The purified chimeric antibody and control antibody were respectively gradient diluted with 2% BSA, starting at 10 µg/ml, 3 times gradient, with 8 concentration points; the blocking solution was then removed, and the diluted antibody solutions were added to the ELISA plates for incubation at 37°C for 2 h. The plates were washed three times with PBST (0.2% Tween-20), 320 µl/well; after the plates were dried, 100 µl of HRP conjugated Goat anti-Rat IgG (Thermo) was added to each well, followed by incubation at 37°C for 1 h. The plates were washed 5 times with PBST (0.2% Tween-20). After the plates were dried, 100 µl of TMB (Huzhou InnoRagents Co., LTD.) color developing substrate was added to each well for reaction at room temperature for 3-5 min; then, 50 µl of 2M H₂SO₄ was added to each well to stop the color development reaction, and OD 450 nm was given by a microplate reader. The original data were imported into the software GraphPad Prism 6 for nonlinear curve fitting, and the EC₅₀ of antibodies binding to human B7-H3-4Ig-His and monkey B7-H3-4Ig-His was calculated respectively. As shown in Table 1 below, the screened antibodies are all capable of binding to human and monkey B7-H3-4Ig-His proteins.

The affinity of anti-human-B7-H3 human antibody to human breast cancer cell MCF-7 was assayed on a flow cytometer (Beckman, model: Cytoflex). The adherent cells were digested with Trypsin-EDTA (0.25%) (Thermo) solution and then counted and adjusted to the cell density of 4.0×10⁶ cells/ml, washed twice with 1% BSA, and re-suspended in 1% BSA solution; the cell suspension was added to 96-well V-bottomed plates with 50 µl/well ( 2×10⁵ cells/well). The candidate antibody was diluted with 1% BSA, starting at a final concentration of 20 µg/ml, 3 times gradient, with a total of 10 concentration points. 50 µl of the diluted antibody was added to the V-bottomed plates containing cells, and incubated at 4°C for 60 min. The cells were washed twice with 1% BSA, and then 50 µl of a diluted secondary antibody was added to each well, well mixed, and incubated at 4°C for 30 min. The cells were washed twice with 1% BSA and then re-suspended in 200 µl of 1% BSA and assayed by FACS. Data processing: The Median PE values were exported, and then imported into the software GraphPad Prism 6 to calculate EC₅₀. The results are shown in Table 1.

**Table 1: Binding affinity of the chimeric antibody to proteins and tumor cell**

| Chimeric antibody | Human B7-H3 EC₅₀ (ng/ml) | Monkey B7-H3 EC₅₀ (ng/ml) | MCF-7 bonded EC₅₀ (ng/ml) |
|---|---|---|---|
| 20G11G6 | 16.5 | 21.33 | 228.2 |

### 3.2 Sequence fishing of anti-human-B7-H3 chimeric antibody

After about 8000 hybridoma cells were grown, the cells were cleaved, and the first cDNA was synthesized by a cDNA reverse transcription kit (Thermo Fisher). VH and VK genes were amplified from cDNA by PCR using primers. PCR products were purified by a DNA purification kit (Qiagen) and linked to a TOPO vector (Thermo Fisher Sci). Approximately 12 clones were sequenced for each conjugation reaction. The sequences were analyzed by IMGT. The variable region sequences and CDR sequences of the anti-human-B7-H3 antibody were obtained, as shown in Table 2.

**Table 2: Amino acid sequences of variable regions and CDRs in the anti-human-B7-H3 antibody**

| Antibody | VH (SEQ ID NO:) | VL (SEQ ID NO: ) | Defined by | CDR1 of heavy chain (SEQ ID NO:) | CDR2 of heavy chain (SEQ ID NO:) | CDR3 of heavy chain (SEQ ID NO:) | CDR1 of light chain (SEQ ID NO:) | CDR2 of light chain (SEQ ID NO:) | CDR3 of light chain (SEQ ID NO:) |
|---|---|---|---|---|---|---|---|---|---|
| 20G11G6 /2# | 1 | 2 | IMGT | 5 | 6 | 7 | 8 | 9 | 10 |
| | | | Chothia | 11 | 12 | 13 | 14 | 15 | 10 |
| | | | Kabat | 16 | 17 | 13 | 14 | 15 | 10 |
| | | | AbM | 26 | 27 | 13 | 14 | 15 | 10 |

### Example 4: Evaluation of anti-human-B7-H3 human antibody

### 4.1 Expression of anti-human-B7-H3 human antibody

The amino acid sequence of the VH of 20G11G6 was linked to the amino acid sequence (SEQ ID NO: 30) of the CH of human IgG1, and the amino acid sequence of the VL was linked to the amino acid sequence (SEQ ID NO: 32) of the constant region of human light chain x, respectively, to construct B7-H3 human antibody 2# and its heavy chain amino acid sequence and light chain amino acid sequence are shown in SEQ ID NO: 40 and SEQ ID NO: 41, respectively. cDNA was synthesized by codon optimization and linked to plasmid pTT5 (done by GenScript). The pTT5 plasmids corresponding to the nucleotides of heavy and light chains in the human antibody were simultaneously transfected into CHO-EBNA cells, and the supernatant was collected by centrifugation. The recombinant antibody in the supernatant was purified using Protein A (MabSelect SuRe, GE) to obtain the anti-human-B7-H3 human antibody 2# protein (hereinafter referred to as "antibody 2#").

### 4.2 Detection of binding affinity of anti-human-B7-H3 human antibody protein

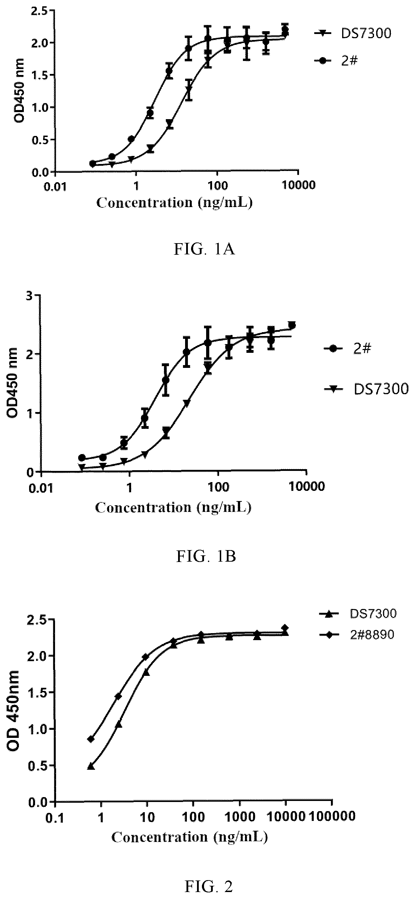

The affinity of the anti-human-B7-H3 human antibody to human B7-H3-4Ig-his and monkey B7-H3-4Ig-his was assayed as follows. Human and monkey B7-H3-4Ig-His proteins were diluted with CBS coating solution to 1 µg/ml, then added to 96-well ELISA plates with 100 µl/well, and incubated overnight at 4°C. The ELISA plates were washed once with PBS and then dried; 100 µl of 2% BSA was then added to each well, followed by incubation at 37°C for 2 h; the blocking solution was then removed. The human antibody and control antibody DS7300 were respectively gradient diluted with 2% BSA, starting at 10 µg/ml, 3 times gradient, with 11 concentration points; and the diluted antibody solutions were added to the ELISA plates with 100 µl/well for incubation at 37°C for 2 h. The plates were washed three times with PBST (0.2% Tween-20); after the plates were dried, 100 µl of HRP Goat anti-human IgG(H+L) (Jackson) was added to each well, followed by incubation at 37°C for 1 h. The plates were washed 5 times with PBST (0.2% Tween-20) and then dried, 100 µl of TMB (Huzhou InnoRagents Co., LTD.) color developing substrate was added to each well for reaction at room temperature for 3-5 min; then, 50 µl of 2M H₂SO₄ was added to each well to stop the color development reaction, and OD₄₅₀ₙₘ was given by a microplate reader. The original data were imported into the software GraphPad Prism 6 for nonlinear curve fitting, and the EC₅₀ was calculated. As shown in Figs. 1A-B and Table 3, antibody 2# has a good binding affinity to human and monkey B7-H3-4Ig.

**Table 3: Binding affinity of anti-human-B7-H3 human antibody**

| Human antibodies | Human B7-H3-4Ig EC₅₀ (ng/ml) | Monkey B7-H3-4Ig EC₅₀ (ng/ml) |
|---|---|---|
| DS7300 | 13.76 | 23.68 |
| 2# | 2.972 | 3.936 |

### 4.3 Determination of isoelectric point (PI) of recombinant human antibody

The isoelectric point (PI) of candidate antibody was determined by isoelectric focusing. The method was briefed as follows: Maurice isoelectric focusing system (ProteinSimple) was used for assay, with the help of capillary cassette. The antibody was diluted with water, and the pH gradient was formed by the test system using amphoteric electrolyte 3-10 (GE ) with a final concentration of 4%. The isoelectric point of the antibody is shown in Table 4.

The results showed that the isoelectric point of antibody 2# was high, about 9.3, so it was necessary to modify the sequence of antibody 2# to reduce the isoelectric point.

**Table 4: Isoelectric point of anti-human-B7-H3 human antibody**

| Human antibody | PI |
|---|---|
| 2# | 9.25 |

### 4.4 Sequence modification of anti-human-B7-H3 human antibody:

In order to reduce the PI of the anti-human-B7-H3 human antibody 2# and remove PTM risk sites, and retain its activity, the VH sequence of antibody 2# was designed with multiple modifications, and the CH of the antibody underwent modifications (L234A, L235A, G237A) to remove ADCC and the amino acid sequence of mutated CH of human IgG1 was SEQ ID NO: 31, and the VL of antibody 2# was designed with multiple modifications to reduce the PI, but the amino acid sequence (SEQ ID NO: 32) of the light chain κ constant region was not modified. The designed antibody was named 2#8890. The variable region sequences and CDR sequences of the anti-human-B7-H3 antibody 2#8890 were obtained, as shown in Table 5. The amino acid sequences of the heavy and light chains of 2#8890 are as shown in SEQ ID NO: 42 and SEQ ID NO: 43, respectively.

**Table 5: Amino acid sequences of variable regions and CDRs in the anti-human-B7-H3 antibody 2#8890**

| Antibody | VH (SEQ ID NO:) | VL (SEQ ID NO: ) | Defined by | CDR1 of heavy chain (SEQ ID NO:) | CDR2 of heavy chain (SEQ ID NO:) | CDR3 of heavy chain (SEQ ID NO:) | CDR1 of light chain (SEQ ID NO:) | CDR2 of light chain (SEQ ID NO:) | CDR3 of light chain (SEQ ID NO:) |
|---|---|---|---|---|---|---|---|---|---|
| 2#8890 | 3 | 4 | IMGT | 18 | 19 | 20 | 8 | 9 | 10 |
| | | | Chothia | 21 | 22 | 23 | 14 | 15 | 10 |
| | | | Kabat | 24 | 25 | 23 | 14 | 15 | 10 |
| | | | AbM | 28 | 29 | 23 | 14 | 15 | 10 |

### 4.5 Expression of anti-human-B7-H3 human antibody

The sequence of 2#8890 was codon-optimized ( by GenScript) to synthesize cDNA which was linked to the plasmid pTT5. The pTT5 plasmids corresponding to the nucleotides of heavy and light chains in the human antibody were simultaneously transfected into CHO-EBNA cells, and the supernatant was collected by centrifugation. The recombinant antibody in the supernatant was purified using Protein A (MabSelect SuRe, GE) to obtain the anti-human-B7-H3 human antibody.

According to the PI determination method described in 4.3, the PI of the antibody 2#8890 was determined, and its PI was reduced to 8.4, successfully reducing the PI of the anti-human-B7-H3 human antibody.

### 4.6 Detection of protein binding affinity of anti-human-B7-H3 human antibody

The affinity of the anti-human-B7-H3 human antibody to human B7-H3-4Ig-his was assayed as follows. Human B7-H3-4Ig-his protein was diluted with CBS coating solution to 1 µg/ml, then added to 96-well ELISA plates with 100 µl/well, and incubated overnight at 4°C. The ELISA plates were washed once with PBS and then dried; 100 µl of 2% BSA was then added to each well, followed by incubation at 37°C for 2 h; the blocking solution was then removed. The human antibody and the control antibody were respectively gradient diluted with 2% BSA, starting at 10 µg/ml, 4 times gradient, with 8 concentration points; and the diluted antibody solutions were added to the ELISA plates with 100 µl/well for incubation at 37°C for 2 h. The plates were washed three times with PBST (0.2% Tween-20); after the plates were dried, 100 µl of HRP Goat anti-human IgG(H+L) (Jackson) was added to each well, followed by incubation at 37°C for 1 h. The plates were washed 5 times with PBST (0.2% Tween-20) and then dried, 100 µl of TMB (Huzhou InnoRagents Co., LTD.) color developing substrate was added to each well for reaction at room temperature for 3-5 min; then, 50 µl of 2 M H₂SO₄ was added to each well to stop the color development reaction, and OD_{450 nm} was given by a microplate reader. The original data were imported into the software GraphPad Prism 6 for nonlinear curve fitting, and the EC₅₀ was calculated. As shown in Table 6 and FIG. 2, antibody 2#8890 has a good binding affinity to B7-H3-4Ig.

**Table 6: Binding affinity of the anti-human-B7-H3 human antibody to human B7-H3-4Ig-His protein**

| Abs | Human B7-H3-4Ig-His EC₅₀ (ng/mL) |
|---|---|
| DS7300 | 3.357 |
| 2#8890 | 1.929 |

### 4.7 Detection of dynamic affinity of anti-human-B7-H3 human antibody

The dynamic affinity of anti-human-B7-H3 antibody to human B7-H3-4Ig-his, human B7-H3-2Ig-his, rat B7-H3-his and monkey B7-H3-his proteins was determined by ForteBio (Pall life sciences). The method was described specifically as follows: the antibody to be tested was diluted with PBST (0.02% Tween-20) to 5 µg/ml, and the human B7-H3-4Ig-his, human B7-H3-2Ig-his, rat B7-H3-his and monkey B7-H3-his proteins were gradient diluted to 200 nM, 100 nM, 50 nm, 25 nM, 12.50 nM, 6.25 nM, 3.125 nM, and 0 nM; then, the antibody to be tested was captured in PBST (0.02% Tween-20) solution using Protein A Sensor (Pall life) separately for 60 s, and then bound to the four proteins for 60 s respectively, and then dissociated for 180 s. The obtained results were opened in the software Data Analysis 11.0, and the 1:1 mode was selected for global fitting to analyze the results and obtain the affinity constant. The results are shown in Table 7. The results showed that 2#8890 was strongly bound to human and monkey B7-H3, but not to rat B7-H3.

**Table 7 Dynamic affinity of anti-human-B7-H3 human antibody**

| Abs | Human B7-H3-4Ig KD (M) | Human B7-H3-2Ig KD (M) | Rat B7-H3 KD (M) | Monkey B7-H3 KD (M) |
|---|---|---|---|---|
| DS7300 | 1.70E-10 | 8.99E-09 | Not bond | 1.99E-09 |
| 2#8890 | 7.83E-10 | 1.83E-08 | Not bond | 1.24E-09 |

### 4.8 Cell affinity and cross-species assay of anti-human-B7-H3 human antibody

The affinity of the anti-human-B7-H3 human antibody to human colon cancer cell HT29 (National Collection of Authenticated Cell Cultures), human gastric cancer cell NCI-N87 (ATCC), human mammary squamous cancer cell HCC1806 (ATCC) and human non-small cell lung cancer cell HCC827 (ATCC) was assayed on a flow cytometer (Beckman, model: Cytoflex); the affinity of the anti-human-B7-H3 human antibody to CHO-S-human B7-H3-4Ig and CHO-S-human B7-H3-2Ig was detected. Species crossover of the anti-human-B7-H3 human antibody was detected: affinity to CHO-S-rat B7-H3 and CHO-S-monkey B7-H3 was detected.

The adherent cells were digested with Trypsin-EDTA (0.25%) (Thermo) solution and then counted and adjusted to the cell density of 4.0×10⁶ cells/ml, washed twice with 1% BSA, and re-suspended in 1% BSA solution; the cell suspension was added to 96-well V-bottomed plates with 50 µl/well ( 2×10⁵ cells/well). The candidate antibody and the negative control antibody hIgG1 were respectively diluted with 1% BSA, starting at a final concentration of 10 µg/ml, 3 times gradient, with a total of 11 concentration points. 50 µl of the diluted antibody was added to the V-bottomed plates containing cells, and incubated at 4°C for 60 min. The cells were washed twice with 1% BSA, and then 50 µl of diluted secondary antibody was added to each well, well mixed, and incubated at 4°C for 30 min. The cells were washed twice with 1% BSA and then re-suspended in 200 µl of 1% BSA and assayed by FACS. Data processing: The Median PE values were exported, and then imported into the software GraphPad Prism 6 to calculate EC₅₀.

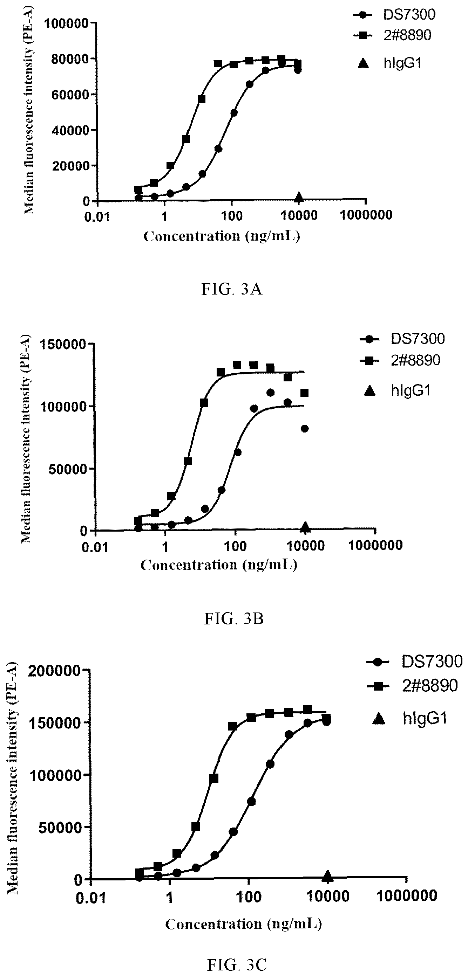

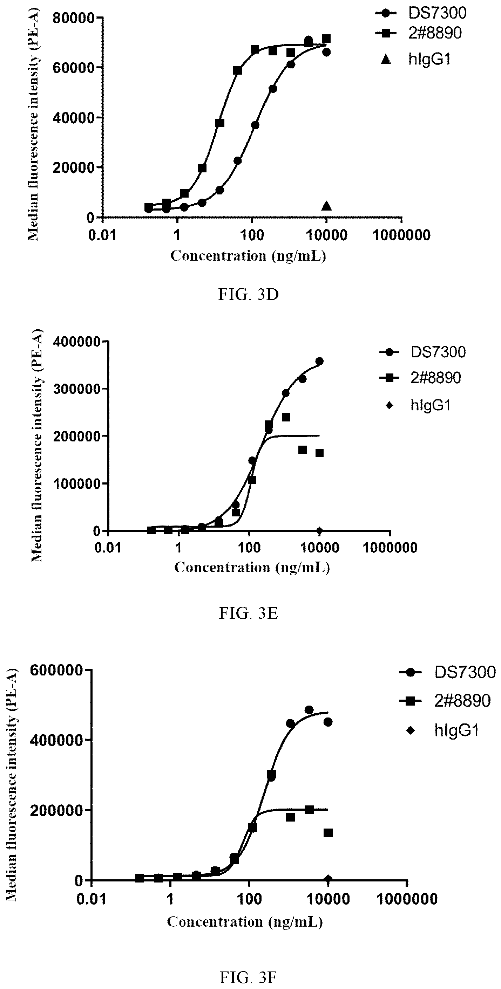

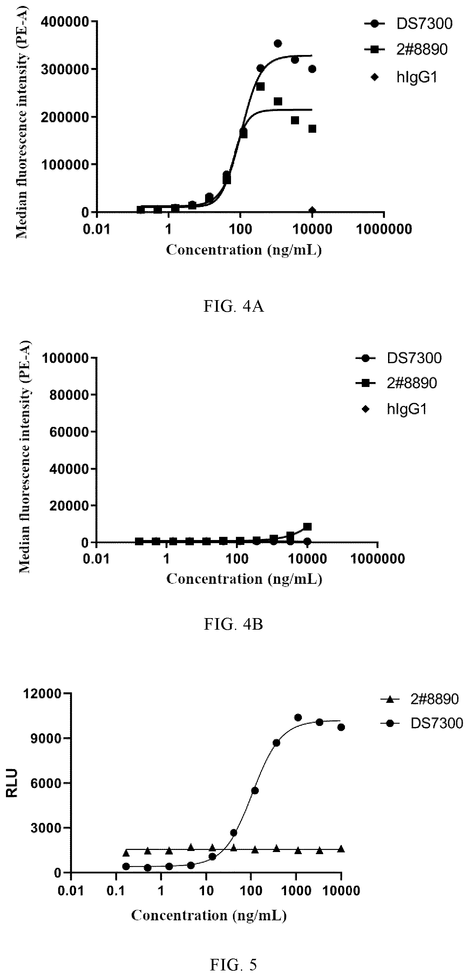

The results of affinity of the anti-human-B7-H3 human antibody to HT29, NCI-N87, HCC1806 and HCC827 tumor cells are shown in Figs. 3A, 3B, 3C and 3D and Table 8, respectively. The affinity of 2#8890 to the tumor cells was higher than that of DS7300. The results of affinity of the anti-human-B7-H3 human antibody to CHO-S-human B7-H3-4Ig and CHO-S-human B7-H3-2Ig cells are shown in Figs. 3E and 3F and Table 9. The affinity of 2#8890 to these two overexpressing cells is similar to that of DS7300. The results of affinity of the anti-human-B7-H3 human antibody to CHO-S-monkey B7-H3 and CHO-S-rat B7-H3 cells are shown in Figs. 4A and 4B and Table 10. The results show that 2#8890 binds to monkey B7-H3-overexpressed cells, but does not bind to rat B7-H3- overexpressed cells.

**Table 8: Cell affinity of the anti-human-B7-H3 human antibody to HT29, NCI-N87, HCC1806 and HCC827**

| Abs | HT29 (ng/ml) | NCI-N87 (ng/ml) | HCC1806 (ng/ml) | HCC827 (ng/ml) |
|---|---|---|---|---|
| DS7300 | 69.45 | 76.95 | 134.4 | 118.9 |
| 2#8890 | 6.336 | 5.772 | 9.666 | 12.27 |

**Table 9: Cell affinity of the anti-human-B7-H3 human antibody to CHO-S-human B7-H3-4Ig and CHO-S-human B7-H3-2Ig**

| Abs | CHO-S-B7-H3-4Ig (ng/ml) | CHO-S-B7-H3-2Ig (ng/ml) |
|---|---|---|
| DS7300 | 228.0 | 243.0 |
| 2#8890 | 115.1 | 68.42 |

**Table 10: Cell affinity of the anti-human-B7-H3 human antibody to CHO-S-rat B7-H3 and CHO-S-monkey B7-H3**

| Abs | CHO-S-rat B7-H3 (ng/ml) | CHO-S-monkey B7-H3 (ng/ml) |
|---|---|---|
| DS7300 | Not bond | 109.1 |
| 2#8890 | Not bond | 64.88 |

### 4.9 Detection of ADCC of anti-human-B7-H3 human antibody

Effector cell Jurkat-NFAT/Luciferase-CD16A (an engineered Jurkat cell that stably expresses CD16a and luciferase reporter genes regulated by NFAT response elements and that is obtained by lentivirus packaging, infection and pressure screening) can activate the expression of luciferase and emit fluorescence signals using a One-Glo reagent (Promega) as a substrate in the presence of both antibodies and target cells. The specific assay was carried out as follows: Jurkat-NFAT/luciferase-CD16a and CHO-S-human B7-H3-4Ig cells (engineered CHO-S cells stably expressing human B7-H3-4Ig and obtained by lentivirus packaging, infection and pressure screening) were collected by centrifugation, and then re-suspended in RPMI 1640+1% FBS medium and adjusted to the density of 2.0×10⁶ cells/ml and 1.0×10⁶ cells/ml respectively, and then added to 96-well plates respectively with 50 µl/well. The antibody was diluted with RPMI 1640+1% FBS medium (starting at 10 µg/ml, 3 times gradient, with 11 concentration points), and then added to 96-well plates with 50 µl/well, followed by incubation at 37 °C for 5 h. 20 µl of One-glo (Promega) reagent was added and well mixed by shaking and then the fluorescence signal value was given by a microplate reader. The results were imported into the software Graph Prism 6 to calculate the EC₅₀. As shown in FIG. 5 and Table 11, 2#8890 has no ADCC, while DS7300 retains ADCC and has EC₅₀ of 108 ng/ml.

**Table 11: ADCC of anti-human-B7-H3 human antibody**

| Abs | EC₅₀(ng/ml) |
|---|---|
| DS7300- | 108.2 |
| 2#8890 | no ADCC |

### 4.10 Detection of CDC of anti-human-B7-H3 human antibody

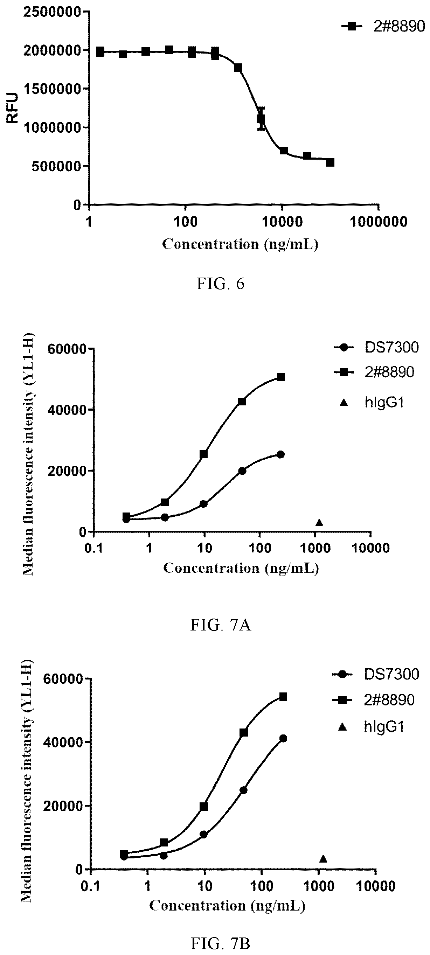

To detect the CDC (complement-dependent cytotoxicity) effect and intensity of the anti-human-B7-H3 human antibody, target cell CHO-S-B7-H3-4Ig was collected and counted, and the cell density was adjusted to 2.5×10⁵ cells/ml using base medium DMEM, and the cell suspension was added to 96-well plates with 40 µl/well (the number of cells was 1×10⁴ per well); a bottle of freeze-dried guinea pig serum complement was dissolved with 1 ml of DMEM base medium such that the complement concentration was 100%; then, 950 µl of 100% complement was well mixed with 950 µl of DMEM such that the complement concentration was 50%; then, 20 µl of 50% guinea pig serum complement was added to 96-well plates such that the final complement concentration in wells was 10%; the diluent of the antibody to be tested was then added with 40 µl/well, where the antibody to be tested with the final concentration of 100,000 ng /ml was diluted 3 times, with 11 concentrations. The 96-well plates were placed in a 37 °C, CO₂ constant-temperature incubator for incubation for 2 h. After incubation, Cell Counting luminescent substrate solution was added to the 96-well plates with 50 µl/well and well mixed by shaking, and then the fluorescence signal value was given by a microplate reader. The results were imported into the software Graph Prism 6 to calculate the EC₅₀. As shown in FIG. 6 and Table 12, 2#8890 has CDC.

**Table 12: CDC of anti-human-B7-H3 human antibody**

| Abs | EC₅₀(ng/ml) |
|---|---|
| 2#8890 | 2917 |

### 4.11 Detection of endocytosis of anti-human-B7-H3 human antibody

The endocytosis of the anti-human-B7-H3 human antibody to human gastric cancer cell NCI-N87 (ATCC), human mammary squamous carcinoma cell HCC1806 (ATCC) and human non-small cell lung cancer cell HCC827(ATCC) was assayed on a flow cytometer (Thermo, model: Attune NxT).

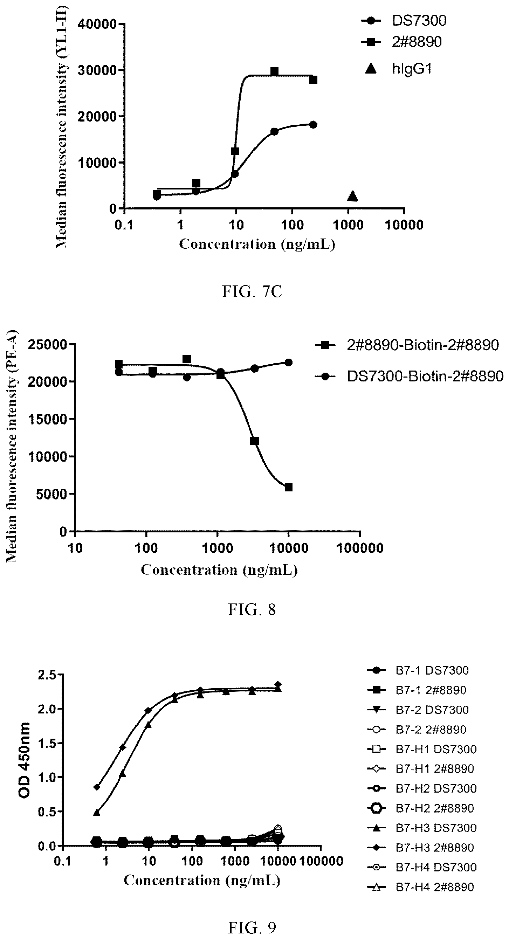

The adherent cells were digested with Trypsin-EDTA (0.25%) (Thermo) solution and counted. The cell density was adjusted to 1×10⁵ cells/ml with a complete medium, and the cell suspension was added to 96-well plates with 100 µl/well (the number of cells was 1×10⁴ per well). The 96-well plates were placed in a 37 °C, CO₂ constant-temperature incubator for incubation for 24 h. The 96-well plates were taken out, the medium was sucked away, and 50 µl of fresh complete medium was added to each well. The antibody to be tested and the negative control antibody hIgG1 were respectively diluted with complete medium, starting at the final concentration of 1.2 µg/ml, 5 times gradient, with a total of 6 concentration points. 300 µg/ml PHrodo reagent (Thermo, Cat#Z25612) was diluted with complete medium to 12 µg/ml (the final concentration of PHrodo was 3 µg/ml). The gradient diluted antibody to be tested was well mixed with the diluted PHrodo reagent at a ratio of 1:1 (30 µl: 30 µl) and incubated in dark at room temperature for 30 min. 50 µl of the mixture of the antibody to be tested and the PHrodo reagent was added to 96-well plates and incubated at 37 °C, 5% CO₂ for 24 h. The 96-well plates were then taken out, the medium was sucked away; the plates were washed once with aseptic PBS; the Trypsin-EDTA (0.25%) was added with 100 µl/well to digest the cells, and 100 µl of complete medium was then added for neutralization. The cells in the wells were blown and dispersed and then assayed by FACS. Data processing: Median APC values were exported and then imported into the software GraphPad Prism 6 to calculate EC₅₀. As shown in Figs. 7A, 7B and 7C and Table 13, the endocytosis of 2#8890 was higher than that of DS7300-.

**Table 13: Endocytosis of anti-human-B7-H3 human antibody**

| Abs | NCI-N87 (ng/ml) | HCC1806 (ng/ml) | HCC827 (ng/ml) |
|---|---|---|---|
| DS7300 | 24.15 | 57.19 | 15.18 |
| 2#8890 | 12.20 | 20.64 | 10.24 |

### 4.12 Antigen-binding epitope analysis of anti-human-B7-H3 human antibody

CHO-S-human B7-H3-4Ig cells grown in suspension were collected, counted and adjusted to the cell density of 4.0×10⁶ cells/ml, washed twice with 1% BSA, and re-suspended in 1% BSA solution. The cell suspension was added to 96-well V-bottomed plates with 50 µl/well (the number of cells was 2×10⁵ per well). Biotin-2#8890 was diluted with 1% BSA to 80 ng/ml (final concentration was 20 ng/ml), and 25 µl of the diluted Biotin-2#8890 was added to the V-bottomed plates with cells. DS7300 and 2#8890 were respectively diluted with 1% BSA, starting at 10 µg/ml, 3 times gradient, with a total of 6 concentration points, and then the diluted antibody solutions, each 25 µl, were added to the 96-well plates separately. The cells, concentration-gradient antibodies and biotin-labelled antibody with constant-concentration were well mixed and incubated at 4°C for 60 min. The cells were then washed twice with 1% BSA, and a diluted PE Streptavidin secondary antibody (Biolegend) was added with 50 µl/ well, followed by incubation at 4°C for 30 min. The cells were then washed twice with 1% BSA, and then re-suspended in 200 µl of PBS for FACS. Data processing: The Median PE values were exported, and then imported into the software GraphPad Prism 6 for nonlinear curve fitting (four parameters). As shown in FIG. 8, 2#8890 has no epitope competition with DS7300.

### 4.13: Detection of specificity of anti-human-B7-H3 human antibody

The specificity of anti-human-B7-H3 human antibody binding to human B7-H3 was assayed as follows. ELISA was carried out to detect the binding affinity of the anti-human-B7-H3 human antibody to human B7-1-his (purchased from Novoprotein Scientific Inc.), B7-2-his (purchased from Novoprotein Scientific Inc.), B7-H1-mFc (self-constructed), B7-H2-his (purchased from Novoprotein Scientific Inc.), B7-H3-4Ig-his and B7-H4-hFc (purchased from Novoprotein Scientific Inc.). Human B7-1-his, B7-2-his, B7-H1-mFc, B7-H2-his, B7-H3-4Ig-his and B7-H4-hFc proteins were respectively diluted with a CBS coating solution to 1 µg/ml, and added to 96-well ELISA plates with 100 µl/well and incubated overnight at 4°C. The plates were washed once with PBS and then dried, and then 2% BSA was added with 100 µl/well for incubation at 37°C for 2 h; then, the blocking solution was removed. Human antibodies 2#8890 and 2# 8890-biotin and control antibodies DS7300 and DS7300-biotin were diluted with 2% BSA, respectively, starting at 10 µg/ml, 4 times gradient, with 8 concentration points. To B7-1-his, B7-2-his, B7-H2-his and B7-H3-4Ig-his, added were 2#8890 and DS7300 with 100 µl/well, respectively; to human B7-H1-mFc and B7-H4-hFc, added were 2#8890-biotin and DS7300-biotin with 100 µl/well. Then, incubation was carried out at 37°C for 2 h. The plates were washed three times with PBST (0.2% Tween-20) and then dried. To B7-1-his, B7-2-his, B7-H2-his and B7-H3-4Ig-his, added was HRP Goat Anti-Human IgG(H+L) (Jackson) diluted at a ratio of 1:10000, with 100 µl/well; to human B7-H1-mFc and B7-H4-hFc, added was peroxidase-conjugated streptavidin secondary antibody (Jackson) diluted a ratio of 1:10000, with 100 µl/well. Then, incubation was carried out at 37°C for 1 h. The plates were washed 5 times with PBST (0.2% Tween-20) and then dried, 100 µl of TMB (Huzhou InnoRagents Co., LTD.) color developing substrate was added to each well for reaction at room temperature for 3-5 min; then, 50 µl of 2M H₂SO₄ was added to each well to stop the color development reaction, and OD₄₅₀ₙₘ was given by the microplate reader. The original data were imported into the software GraphPad Prism 6 for nonlinear curve fitting, and the EC₅₀ was calculated. As shown in Table 14 and FIG. 9, 2#8890 was specific for binding to B7-H3-4Ig, while 2#8890 does not bind to B7-1, B7-2, B7-H1, B7-H2 and B7-H4.

**Table 14: Specificity of anti-human-B7-H3 human antibody**

| Abs | Human B7-1 (ng/ml) | Human B7-2 (ng/ml) | Human B7-H1 (ng/ml) | Human B7-H2 (ng/ml) | Human B7-H3 (ng/ml) | Human B7-H4 (ng/ml) |
|---|---|---|---|---|---|---|
| DS7300 | Not bond | Not bond | Not bond | Not bond | 3.357 | Not bond |
| 2#8890 | Not bond | Not bond | Not bond | Not bond | 1.929 | Not bond |

Although specific embodiments of the invention have been described in detail, those skilled in the art should understand that various modifications and changes may be made to the details in accordance with all the published teachings, and all modifications and changes are also within the scope of the invention. The scope of the invention is defined by the appended claims and any equivalents thereof.

## Claims

1. An antibody specifically binding to B7-H3 or an antigen-binding fragment thereof, wherein the antibody or the antigen-binding fragment thereof comprises the following complementary determining regions (CDRs):
(a) a CDR-H1, a CDR-H2 and a CDR-H3 contained in a heavy chain variable region (VH) as set forth in SEQ ID NO: 1; and/or a CDR-L1, CDR-L2 and CDR-L3 contained in a light chain variable region (VL) as set forth in SEQ ID NO: 2;
(b) a CDR-H1, a CDR-H2 and a CDR-H3 contained in a heavy chain variable region (VH) as set forth in SEQ ID NO: 3; and/or a CDR-L1, CDR-L2 and CDR-L3 contained in a light chain variable region (VL) as set forth in SEQ ID NO: 4; or
(c) a CDR-H1, a CDR-H2 and a CDR-H3 contained in the following heavy chain variable region (VH), and/or a CDR-L1, a CDR-L2 and a CDR-L3 contained in the following light chain variable region (VL), wherein the heavy chain variable region (VH) and/or the light chain variable region (VL) contains a mutation in at least one CDR compared to the heavy chain variable region and/or the light chain variable region described in (a) or (b), wherein the mutation is a substitution, deletion, or addition of one or more amino acids (for example, a substitution, deletion, or addition of 1, 2 or 3 amino acids); preferably, the substitution is a conservative substitution.
preferably, the CDRs are defined according to the IMGT, Kabat, Chothia, or AbM numbering system.

2. The antibody or an antigen-binding fragment thereof according to claim 1, wherein the antibody or the antigen-binding fragment thereof comprises:
(1) the following heavy chain variable region (VH) and/or light chain variable region (VL), wherein CDRs are defined by the IMGT numbering system:
(1a) a heavy chain variable region (VH) comprising the following three CDRs: a CDR-H1 with a sequence as set forth in SEQ ID NO: 5 or a variant thereof; a CDR-H2 with a sequence as set forth in SEQ ID NO: 6 or a variant thereof; and a CDR-H3 with a sequence as set forth in SEQ ID NO: 7 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
(1b) a heavy chain variable region (VH) comprising the following three CDRs: a CDR-H1 with a sequence as set forth in SEQ ID NO: 18 or a variant thereof; a CDR-H2 with a sequence as set forth in SEQ ID NO: 19 or a variant thereof; and a CDR-H3 with a sequence as set forth in SEQ ID NO: 20 or a variant thereof; and/or, a light chain variable region (VL) comprising the following three CDRs: a CDR-L1 with a sequence as set forth in SEQ ID NO: 8 or a variant thereof; a CDR-L2 with a sequence as set forth in SEQ ID NO: 9 or a variant thereof; and a CDR-L3 with a sequence as set forth in SEQ ID NO: 10 or a variant thereof;
or,
(2) the following heavy chain variable region (VH) and/or light chain variable region (VL), wherein CDRs are defined by the Chothia numbering system:
(2a) a heavy chain variable region (VH) comprising the following three CDRs: a CDR-H1 with a sequence as set forth in SEQ ID NO: 11 or a variant thereof; a CDR-H2 with a sequence as set forth in SEQ ID NO: 12 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(2b) a heavy chain variable region (VH) comprising the following three CDRs: a CDR-H1 with a sequence as set forth in SEQ ID NO: 21 or a variant thereof; a CDR-H2 with a sequence as set forth in SEQ ID NO: 22 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
or,
(3) the following heavy chain variable region (VH) and/or light chain variable region (VL), wherein CDRs are defined by the Kabat numbering system:
(3a) a heavy chain variable region (VH) comprising the following three CDRs: a CDR-H1 with a sequence as set forth in SEQ ID NO: 16 or a variant thereof; a CDR-H2 with a sequence as set forth in SEQ ID NO: 17 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(3b) a heavy chain variable region (VH) comprising the following three CDRs: a CDR-H1 with a sequence as set forth in SEQ ID NO: 24 or a variant thereof; a CDR-H2 with a sequence as set forth in SEQ ID NO: 25 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
or,
(4) the following heavy chain variable region (VH) and/or light chain variable region (VL), wherein CDRs are defined by the AbM numbering system:
(4a) a heavy chain variable region (VH) comprising the following three CDRs: a CDR-H1 with a sequence as set forth in SEQ ID NO: 26 or a variant thereof; a CDR-H2 with a sequence as set forth in SEQ ID NO: 27 or a variant thereof; and a CDR-H3 with a sequence as set forth in SEQ ID NO: 13 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
(4b) a heavy chain variable region (VH) comprising the following three CDRs: a CDR-H1 with a sequence as set forth in SEQ ID NO: 28 or a variant thereof; a CDR-H2 with a sequence as set forth in SEQ ID NO: 29 or a variant thereof; and a CDR-H3 with a sequence as set forth in SEQ ID NO: 23 or a variant thereof; and/or, a light chain variable region (VL) comprising the following three CDRs: a CDR-L1 with a sequence as set forth in SEQ ID NO: 14 or a variant thereof; a CDR-L2 with a sequence as set forth in SEQ ID NO: 15 or a variant thereof; and a CDR-L3 with a sequence as set forth in SEQ ID NO: 10 or a variant thereof;
wherein the variant in any one of (1a), (1b), (2a), (2b), (3a), (3b), (4a) and (4b) has a substitution, deletion or addition of one or more amino acids (for example, a substitution, deletion or addition of 1, 2 or 3 amino acids) compared to the sequence from which the variant is derived; preferably, the substitution is a conservative substitution.

3. The antibody or an antigen-binding fragment thereof according to claim 1 or 2, wherein the antibody or the antigen-binding fragment thereof comprises:
(a) a VH containing a sequence as set forth in SEQ ID NO: 1 or a variant thereof; and/or a VL containing a sequence as set forth in SEQ ID NO: 2 or a variant thereof; or
(b) a VH containing a sequence as set forth in SEQ ID NO: 3 or a variant thereof; and/or a VL containing a sequence as set forth in SEQ ID NO: 4 or a variant thereof;
wherein the variant has at least 80%, at least 85%, at least 90%, at least 91%, at least 92%, at least 93%, at least 94%, at least 95%, at least 96%, at least 97%, at least 98%, at least 99%, or 100% sequence identity to the sequence from which the variant is derived or has a substitution, deletion or addition of one or more amino acids (for example, a substitution, deletion or addition of 1, 2, 3, 4 or 5 amino acids) compared to the sequence from which the variant is derived; preferably, the substitution is a conservative substitution.

4. The antibody or an antigen-binding fragment thereof according to any one of claims 1-3, wherein the antibody or the antigen-binding fragment thereof is a murine antibody, a chimeric antibody, a humanized antibody, or a human antibody.

5. The antibody or an antigen-binding fragment thereof according to any one of claims 1-4, wherein the antibody or the antigen-binding fragment thereof further comprises a constant region from or derived from a human immunoglobulin;
preferably, a heavy chain of the antibody or the antigen-binding fragment thereof comprises a heavy chain constant region from or derived from a human immunoglobulin, such as IgG1, IgG2, IgG3 or IgG4; preferably, the antibody or the antigen-binding fragment thereof comprises a wild-type Fc region, or comprises a mutated or chemically modified Fc region that has altered effector function as compared to the wild-type Fc region;
preferably, the antibody or the antigen-binding fragment thereof comprises a variant of the heavy chain constant region of human IgG1, wherein the variant has the following substitutions as compared to a wild-type sequence from which the variant is derived: Leu234Ala, Leu235Ala, and Gly237Ala (according to the locations in the EU numbering system);
preferably, a light chain of the antibody or the antigen-binding fragment thereof comprises a light chain constant region from or derived from a human immunoglobulin, such as κ or λ;
preferably, the antibody or the antigen-binding fragment thereof comprises a heavy chain constant region (CH) as set forth in SEQ ID NO: 30 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to SEQ ID NO: 30;
preferably, the antibody or the antigen-binding fragment thereof comprises a heavy chain constant region (CH) as set forth in SEQ ID NO: 31 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to SEQ ID NO: 31;
preferably, the antibody or the antigen-binding fragment thereof comprises a light chain constant region (CL) as set forth in SEQ ID NO: 32 or a variant thereof, wherein the variant comprises conservative substitutions of up to 20 amino acids (e.g., conservative substitutions of up to 15, up to 10, or up to 5 amino acids; e.g., conservative substitutions of 1, 2, 3, 4 or 5 amino acids) as compared to SEQ ID NO: 32;
more preferably, the antibody or the antigen-binding fragment thereof comprises a heavy chain constant region (CH) as set forth in SEQ ID NO: 30 and a light chain constant region (CL) as set forth in SEQ ID NO: 32; or, the antibody or the antigen-binding fragment thereof comprises a heavy chain constant region (CH) as set forth in SEQ ID NO: 31 and a light chain constant region (CL) as set forth in SEQ ID NO: 32.

6. The antibody or the antigen-binding fragment thereof according to claim 5, wherein the heavy chain constant region (CH) or a variant thereof as set forth in SEQ ID NO: 30 or 31 lacks C-terminal lysine.

7. The antibody or an antigen-binding fragment thereof according to any one of claims 1-6, wherein the antibody or the antigen-binding fragment thereof comprises:
(1) a heavy chain containing a VH as set forth in SEQ ID NO: 1 and a heavy chain constant region (CH) as set forth in SEQ ID NO: 30; and a light chain containing a VL as set forth in SEQ ID NO: 2 or a light chain constant region (CL) as set forth in SEQ ID NO: 32;
(2) a heavy chain containing a VH as set forth in SEQ ID NO: 3 and a heavy chain constant region (CH) as set forth in SEQ ID NO: 30; and a light chain containing a VL as set forth in SEQ ID NO: 4 or a light chain constant region (CL) as set forth in SEQ ID NO: 32;
(3) a heavy chain containing a VH as set forth in SEQ ID NO: 1 and a heavy chain constant region (CH) as set forth in SEQ ID NO: 31; and a light chain containing a VL as set forth in SEQ ID NO: 2 or a light chain constant region (CL) as set forth in SEQ ID NO: 32; or
(4) a heavy chain containing a VH as set forth in SEQ ID NO: 3 and a heavy chain constant region (CH) as set forth in SEQ ID NO: 31; and a light chain containing a VL as set forth in SEQ ID NO: 4 or a light chain constant region (CL) as set forth in SEQ ID NO: 32.

8. The antibody or the antigen-binding fragment thereof according to claim 3 or 7, wherein the N-terminal glutamine of the VH having the sequence as set forth in SEQ ID NO: 1 or 3 or a variant thereof undergoes cyclization to form pyroglutamic acid or pyroglutamate; and/or
the N-terminal glutamic acid of the VL having the sequence as set forth in SEQ ID NO: 2 or 4 or variants thereof undergoes cyclization to form pyroglutamic acid or pyroglutamate.

9. The antibody or an antigen-binding fragment thereof according to any one of claims 1-8, wherein the antibody or the antigen-binding fragment thereof comprises:
(1) a heavy chain having a sequence as set forth in SEQ ID NO: 40 and a light chain having a sequence as set forth in SEQ ID NO: 41; or
(2) a heavy chain having a sequence as set forth in SEQ ID NO: 42 and a light chain having a sequence as set forth in SEQ ID NO: 43.

10. The antibody or the antigen-binding fragment thereof according to claim 9, wherein the N-terminal glutamine of the heavy chain having the sequence as set forth in SEQ ID NO: 40 or 42 or a variant thereof undergoes cyclization to form pyroglutamic acid or pyroglutamate; and/or
the N-terminal glutamic acid of the light chain having the sequence as set forth in SEQ ID NO: 41 or 43 or variants thereof undergoes cyclization to form pyroglutamic acid or pyroglutamate.

11. The antibody or an antigen-binding fragment thereof according to any one of claims 1-10, wherein the antibody or the antigen-binding fragment thereof is selected from the group consisting of ScFv, Fab, Fab ', Fab'-SH, F(ab')2, Fv fragment, disulfide-stabilized Fv(dsFv), diabody, bispecific antibody, and multispecific antibody.

12. The antibody or an antigen-binding fragment thereof according to any one of claims 1-11, wherein the antibody or the antigen-binding fragment thereof carries a label; preferably, the antibody or the antigen-binding fragment thereof carries a detectable label, such as an enzyme (e.g., horseradish peroxidase), radionuclide, fluorescent dye, luminescent substance (e.g., chemiluminescent substance), or biotin.

13. The antibody or the antigen-binding fragment thereof according to any one of claims 1-12, wherein the antibody or the antigen-binding fragment thereof has properties selected from the following:
(1) binding to B7-H3 (e.g., human or monkey B7-H3) with EC50 of less than about 100 ng/mL, such as less than about 80 ng/mL, 50 ng/mL, 20 ng/mL, 15 ng/mL, 14 ng/mL, 13 ng/mL, 12 ng/mL, 11 ng/mL, 10 ng/mL, 9 ng/mL, 8 ng/mL, 7 ng/mL, 6 ng/mL, 5 ng/mL, 4 ng/mL or less; preferably, the EC50 is measured by ELISA;
(2) binding to B7-H3 (e.g., human or monkey B7-H3) with KD of less than about 100 nM, such as less than about 90 nM, 80 nM, 70 nM, 60 nM, 50 nM, 40 nM, 30 nM, 20 nM, 15 nM, 10 nM, 5 nM or less; preferably, the KD is measured by Bio-Layer Interferometry (BLI) (e.g., ForteBio Octet^{®});
(3) not binding to or substantially not binding to B7-1, B7-2, B7-H1, B7-H2 and/or B7-H4, which is assayed by, for example, ELISA;
(4) having CDC, such as inducing killing of cells expressing B7-H3 (e.g., tumor cells) through CDC;
(5) not having ADCC;
(6) inducing endocytosis of B7-H3, which is assayed by, for example, flow cytometry;
(7) inhibiting proliferation of a cell (e.g., a tumor cell); and/or
(8) inhibiting tumor growth.

14. An isolated nucleic acid molecule, comprising a nucleotide sequence encoding the antibody or the antigen-binding fragment thereof according to any one of claims 1-13, a heavy chain and/or light chain, or a heavy chain variable region and/or a light chain variable region thereof.

15. The isolated nucleic acid molecule according to claim 14, comprising a nucleic acid molecule encoding the VH of the antibody, and/or a nucleic acid molecule encoding the VL of the antibody, wherein
(a) the nucleic acid molecule encoding the VH of the antibody comprises: (i) a nucleotide sequence as set forth in SEQ ID NO: 33, (ii) a sequence substantially identical to SEQ ID NO: 33 (e.g., a sequence having at least about 85%, 90%, 95%, 99% or higher sequence identity, or a sequence with one or more nucleotide substitutions, as compared to SEQ ID NO: 33), or (iii) a degenerate sequence of the sequence in (i) or (ii); and/or, the nucleic acid molecule encoding the VL of the antibody comprises: (iv) a nucleotide sequence as set forth in SEQ ID NO: 34, (v) a sequence substantially identical to SEQ ID NO: 34 (e.g., a sequence having at least about 85%, 90%, 95%, 99% or higher sequence identity or a sequence having one or more nucleotide substitutions, as compared to SEQ ID NO: 34), or (vi) a degenerate sequence of the sequence in (iv) or (v);
or,
(b) the nucleic acid molecule encoding the VH of the antibody comprises: (i) a nucleotide sequence as set forth in SEQ ID NO: 35, (ii) a sequence substantially identical to SEQ ID NO: 35 (e.g., a sequence having at least about 85%, 90%, 95%, 99% or higher sequence identity, or a sequence with one or more nucleotide substitutions, as compared to SEQ ID NO: 35), or (iii) a degenerate sequence of the sequence in (i) or (ii); and/or, the nucleic acid molecule encoding the VL of the antibody comprises: (iv) a nucleotide sequence as set forth in SEQ ID NO: 36, (v) a sequence substantially identical to SEQ ID NO: 36 (e.g., a sequence having at least about 85%, 90%, 95%, 99% or higher sequence identity or a sequence having one or more nucleotide substitutions, as compared to SEQ ID NO: 36), or (vi) a degenerate sequence of the sequence in (iv) or (v).

16. A vector, comprising the isolated nucleic acid molecule according to any one of claims 14-15; preferably, the vector is a cloning vector or an expression vector.

17. A host cell, comprising the isolated nucleic acid molecule according to any one of claims 14-15 or the vector according to claim 13.

18. A method for preparing the antibody or the antigen-binding fragment thereof according to any one of claims 1-13, comprising culturing the host cell according to claim 14 under conditions that allow the expression of the antibody or the antigen-binding fragment thereof, and harvesting the antibody or the antigen-binding fragment thereof from the culture of the host cell.

19. A conjugate, comprising the antibody or the antigen-binding fragment thereof according to any one of claims 1-13 and a conjugating moiety linked thereto;
preferably, the conjugating moiety is selected from detectable labels (such as a radioisotope, a fluorescent substance, a luminescent substance, a colored substance, or an enzyme.) or therapeutic agents (such as a cytotoxic agent, a cytokine, a cytotoxin, or a radionuclide.)

20. A multispecific antibody, comprising the antibody or the antigen-binding fragment thereof according to any one of claims 1-13;
preferably, the multispecific antibody comprises the antibody or the antigen-binding fragment thereof according to any one of claims 1-13 as a first antigen-binding domain, and also comprises at least one second antigen-binding domain for other targets;
preferably, the multispecific antibody is a bispecific antibody or a tri-specific antibody or a quad-specific antibody.

21. A chimeric antigen receptor, comprising the antibody or the antigen-binding fragment thereof (e.g., ScFv) according to any one of claims 1-13, a transmembrane domain, and one or more intracellular T cell signal domains.

22. A pharmaceutical composition, comprising the antibody or the antigen-binding fragment thereof according to any one of claims 1-13, the isolated nucleic acid molecule according to claim 14 or 15, or the vector according to claim 16, or the host cell according to claim 17, or the conjugate according to claim 19, or the multispecific antibody according to claim 20, or the chimeric antigen receptor or a host cell expressing the chimeric antigen receptor according to claim 21, and a pharmaceutically acceptable carrier and/or excipient;
preferably, the pharmaceutical composition may also comprise an additional pharmaceutical active agent;
preferably, the additional pharmaceutical active agent is a medicament with antitumor activity;
preferably, the additional pharmaceutical active agent is selected from the group consisting of a B7-H3 inhibitor, an EGFR inhibitor, an HER2 inhibitor, an HER3 inhibitor, an HER4 inhibitor, an IGFR-1 inhibitor, an mTOR inhibitor, a PI3 kinase inhibitor, a c-met or VEGF inhibitor, a chemotherapeutic agent, or any combination thereof;
preferably, the antibody or the antigen-binding fragment thereof and the additional pharmaceutical active agent are provided alone or in combination.

23. A diagnostic or therapeutic kit comprising the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor or a host cell expressing the chimeric antigen receptor according to claim 21, or the pharmaceutical composition according to claim 22, and optionally an instruction for use and/or a drug administration device.

24. Use of the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor or a host cell expressing the chimeric antigen receptor according to claim 21, or the pharmaceutical composition according to claim 22 in preparation of a medicament for inhibiting proliferation of a cell (such as a cell expressing B7-H3, such as a tumor cell) or for prevention and/or treatment and/or adjuvant therapy of a tumor.
preferably, the antibody or the antigen-binding fragment thereof, the isolated nucleic acid molecule, the vector, the host cell, the conjugate, the multispecific antibody, or the pharmaceutical composition are administered in combination with an additional pharmaceutically active agent, e.g., simultaneously, separately, or sequentially;
preferably, the additional pharmaceutical active agent is a medicament with antitumor activity;
preferably, the additional pharmaceutical active agent is selected from the group consisting of a B7-H3 inhibitor, an EGFR inhibitor, a HER2 inhibitor, a HER3 inhibitor, a HER4 inhibitor, an IGFR-1 inhibitor, an mTOR inhibitor, a PI3 kinase inhibitor, a c-met or VEGF inhibitor, a chemotherapeutic drug, or any combination thereof.

25. The use according to claim 24, wherein the tumor is a B7-H3 positive tumor;
preferably, the tumor is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

26. A method for inhibiting proliferation of a cell, comprising contacting the cell with the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor or a host cell expressing the chimeric antigen receptor according to claim 21, or the pharmaceutical composition according to claim 22;
preferably, the cell is a cell expressing B7-H3, such as a tumor cell.

27. A method for prevention and/or treatment and/or adjuvant therapy of a tumor in a subject, the method comprises administration of an effective amount of the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20, or the chimeric antigen receptor or a host cell expressing the chimeric antigen receptor according to claim 21, or the pharmaceutical composition according to claim 22 to a subject in need.

28. The method according to claim 27, further comprising administration of a second therapy to the subject, wherein the second therapy is selected from the group consisting of surgery, chemotherapy, radiotherapy, immunotherapy, gene therapy, DNA therapy, RNA therapy, nanotherapy, viral therapy, adjuvant therapy and any combination thereof;
alternatively, the second therapy may be applied simultaneously, separately, or sequentially with the method according to claim 27.

29. The method according to claim 27 or 28, wherein the tumor is a B7-H3 positive tumor;
preferably, the tumor is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.

30. A method for detecting presence or level of B7-H3 in a sample, comprising contacting the sample with the antibody or the antigen-binding fragment thereof according to any one of claims 1-13 under conditions allowing formation of a complex of the antibody or the antigen-binding fragment thereof and B7-H3, and detecting the formation of the complex;
preferably, the method is used for diagnosing a tumor, for example a B7-H3-positive tumor, such as breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, and thyroid cancer or any combination thereof;
preferably, the method comprises detecting expression level of B7-H3 in a sample to be tested from a subject; and comparing the expression level to a reference value, wherein an increased expression level compared to the reference value is indicative of a tumor.

31. Use of the antibody or the antigen-binding fragment thereof according to any one of claims 1-13, or the isolated nucleic acid molecule according to claim 14 or 15, or the vector according to claim 16, or the host cell according to claim 17, or the conjugate according to claim 19, or the multispecific antibody according to claim 20 in preparation of a kit, wherein the kit is used for detecting presence or level of B7-H3 in a sample and/or for diagnosing a tumor;
preferably, the tumor is a B7-H3 positive tumor;
preferably, the tumor is selected from the group consisting of breast cancer, colorectal cancer, head and neck cancer, renal clear cell carcinoma, renal papillary cell carcinoma, liver cancer, lung adenocarcinoma, lung squamous cell carcinoma, prostate cancer, gastric adenocarcinoma, thyroid cancer, or any combination thereof.
